# EUROPEAN PATENT SPECIFICATION

(11) **EP 2 185 091 B1**
(45) Date of publication and mention of the grant of the patent: **23.05.2012**
(21) Application number: 08799128.7
(22) Date of filing: 04.09.2008
(51) Int. Cl.: A61B 17/70

(54) **MULTI-AXIAL BONE ANCHOR ASSEMBLY**
MEHRACHSIGE KNOCHENANKERANORDNUNG
ENSEMBLE D'ANCRAGE OSSEUX POLYAXIAL

(30) Priority: 06.09.2007 US 851230
(43) Date of publication of application: 19.05.2010
(73) Proprietor: Warsaw Orthopedic, Inc., Warsaw, IN 46581 (US)
(72) Inventor: FARRIS, Robert A., Cordova, Tennessee 38018 (US); MAY, Jason M., Cordova, Tennessee 38016 (US)
(74) Representative: Viering, Jentschura & Partner
(86) International application number: PCT/US2008/075171
(87) International publication number: WO 2009/032871

(56) References cited:
- EP-A- 0 836 835
- WO-A-00/72770
- WO-A-2006/116437
- WO-A-2007/038350
- WO-A-2007/082019
- WO-A-2008/057551
- US-A1- 2007 118 123

## Description

### BACKGROUND

The present disclosure generally relates to orthopedic implants used for correction of spinal injuries and/or deformities, and more specifically, but not exclusively, concerns apparatuses for fixing a portion of the spine to allow correction and/or healing thereof. In some embodiments, the present disclosure is directed to improved apparatus, systems, and assemblies for securing orthopedic implants to bone and, in some embodiments, to vertebrae.

Elongated connecting elements, such as rods, plates, tethers, wires, cables, and other devices have been implemented along the spinal column and connected between two or more anchors engaged between one or more spinal motion segments. Such connecting elements may be rigid and resist movement of the spinal motion segment in response to spinal loading or motion of the spinal motion segment. Other connecting elements are flexible to permit at least some limited spinal motion while providing resistance to loading and motion of the spinal motion segment. Typically, the connecting elements are secured to the spinal column by bone anchors, such as bone screws, that are attached to the vertebrae. While prior bone anchors and screws have been satisfactory for their intended purposes, they have not been satisfactory in all respect.

Therefore, there remains a need for improved apparatus, systems, and assemblies for securing orthopedic implants to bone.

WO 00/72770 discloses a coupling device for connection to a member, comprising: a coupling element defining a longitudinal axis and a passage extending therethrough; and a compression element of a shape-memory material and being disposed about said coupling element, said compression element having a first configuration and a second configuration, said second configuration contracting about said coupling element and compressing a side wall of the passage against the member to thereby limit movement of the member relative to said coupling element.

EP 0 836 835 A, WO 2008/057551 A, US 2007/118123A1, and WO 2006/116437A disclose further bone anchor systems.

### SUMMARY

The present disclosure provides improved apparatus, systems, and assemblies for securing orthopedic implants to bone.

To this end, the invention provides a bone anchor assembly according to claim 1. Further embodiments of the invention are described in the dependent claims.

In one aspect, the present disclosure provides a bone anchor assembly. The bone anchor assembly comprises a bone anchor having a head portion and a bone engaging portion; a crown member shaped and sized to mate with the head portion of the bone anchor; a saddle having an upper portion and a lower portion; and a sleeve-configured for positioning around a portion of the saddle. The upper portion of the saddle includes a channel for receiving an elongated member and a threaded portion for receiving a

WO 2008/057551 discloses a bone anchor assembly comprising a bone anchor having a head portion and a bone engaging portion; a crown member shaped and sized to mate with the head portion of the bone anchor; a saddle having an upper portion and lower portion, the upper portion having a channel for receiving an elongated member and a threaded portion for receiving a compression member, the lower portion configured to receive the crown member and the head portion of the bone anchor therein, the lower portion including a plurality of slots rendering the lower portion outwardly flexible to receive the head portion of the bone anchor and inwardly flexible to secure the head portion of the bone anchor therein; and a sleeve configured for positioning around the lower portion of the saddle, the sleeve sized such that the lower portion of the saddle secures the head portion of the bone anchor therein upon advancement of the sleeve upwardly around the lower portion of the saddle, the sleeve thereby preventing the lower portion of the saddle from flexing outwardly; wherein the bone anchor is moveable with respect to the saddle wherein the bone anchor is secured in a fixed position relative to the saddle by compression of the head portion of the bone anchor between the crown member and lower portion of the saddle created by threadingly advancing the compression member against the elongated member.

compression member. The lower portion of the saddle is configured to receive the crown member and the head portion of the bone anchor therein. The lower portion includes a plurality of slots rendering the lower portion outwardly flexible to receive the head portion of the bone anchor and inwardly flexible to secure the head portion of the bone anchor therein. The sleeve is configured for positioning around the lower portion of the saddle and sized such that the lower portion of the saddle is prevented from flexing outwardly upon advancement of the sleeve upwardly around the lower portion of the saddle, thereby securing the head portion of the bone anchor therein. The bone anchor is moveable with respect to the saddle when the sleeve is positioned around the lower portion. The bone anchor is secured in a fixed position relative to the saddle by compression of the head portion of the bone anchor between the crown member and the lower portion of the saddle. In some embodiments, the lower portion further comprises a seat portion for interfacing with the head portion of the bone anchor. In some embodiments the seat portion is configured to transmit forces from the head portion of the bone anchor radially outward to the sleeve. In some embodiments, the bone anchor is secured in the fixed position relative to the saddle by compression of the head portion of the bone anchor between the crown member and the lower portion of the saddle created by threadingly advancing a compression member against the elongated member. In some embodiments, the lower portion of the saddle and/or the sleeve includes a plurality of cutout portions configured to allow an increased range of motion between the saddle and the bone anchor.

In another aspect, the present disclosure provides a system for orthopedic implantation. The system comprises an elongated member, a compression member, and a bone anchor assembly. The bone anchor assembly comprises a bone anchor having a head portion and a bone engaging portion; a crown member shaped and sized to mate with the head portion of the bone anchor; a saddle having an upper portion and a lower portion; and a sleeve configured for positioning around the saddle. The upper portion of the saddle includes a channel for receiving the elongated member and a threaded portion for receiving the compression member. The lower portion of the saddle is configured to receive the crown member and the head portion of the bone anchor therein. The lower portion includes a plurality of slots rendering the lower portion flexible to receive and retain the head portion of the bone anchor. The lower portion also includes a seat portion for interfacing with the head portion of the bone anchor. The seat portion is an edge at least partially defined by a conically tapered surface. The sleeve is configured for positioning around the lower portion of the saddle and sized such that the lower portion of the saddle is prevented from flexing outwardly upon advancement of the sleeve upwardly around the lower portion of the saddle, thereby securing the head portion of the bone anchor therein. The bone anchor is moveable with respect to the saddle when the sleeve is positioned around the lower portion. The bone anchor is secured in a fixed position relative to the saddle by compression of the head portion of the bone anchor between the crown member and the lower portion of the saddle created by threadingly advancing the compression member against the elongated member. Generally, the lower portion of the saddle is flexible enough to allow entry of the head of the bone anchor and/or the crown, while the sleeve has sufficient strength to prevent outward movement of the lower portion of the saddle to retain the head portion within the saddle in both pivotable and locked positions.

Further aspects, forms, embodiments, objects, features, benefits, and advantages of the present disclosure shall become apparent from the detailed drawings and descriptions provided herein.

### BRIEF DESCRIPTION OF THE DRAWINGS

Fig. 1 is a diagrammatic perspective view of a system according to one aspect of the present disclosure.
Fig. 2 is a diagrammatic perspective cross-section view of the system of Fig. 1.
Fig. 3 is a diagrammatic perspective exploded view of the system of Figs. 1 and 2.
Fig. 4 is a diagrammatic side view of a saddle according to one aspect of the present disclosure.
Fig. 5 is a diagrammatic top view of the saddle of Fig. 4.
Fig. 6 is a diagrammatic bottom view of the saddle of Figs. 4 and 5.
Fig. 7 is a diagrammatic cross-sectional view of the saddle of Figs. 4, 5, and 6 taken along section line 7-7.
Fig. 8 is a diagrammatic top view of a crown washer according to one aspect of the present disclosure.
Fig. 9 is a diagrammatic cross-sectional view of the crown washer of Fig. 8 taken along section line 9-9.
Fig. 10 is a diagrammatic side view of a bone screw according to one aspect of the present disclosure.
Fig. 11 is a diagrammatic bottom view of a sleeve according to one aspect of the present disclosure.
Fig. 12 is a diagrammatic cross-sectional view of the sleeve of Fig. 11 taken along section line 12-12.
Fig. 13 is a diagrammatic perspective view of a bone anchor assembly according to one aspect of the present disclosure.
Fig. 14 is a diagrammatic front cross-sectional view of the bone anchor assembly of Fig. 13.
Fig. 15 is a diagrammatic side view of the bone anchor assembly of Figs. 13 and 14 engaged with a rod and set screw according to one aspect of the present disclosure.
Fig. 16 is a diagrammatic front cross-sectional view of the bone anchor assembly, rod, and set screw of Fig. 15.
Fig. 17 is a diagrammatic side view of the bone anchor assembly, rod, and set screw of Figs. 15 and 16 in a locked position according to one aspect of the present disclosure.

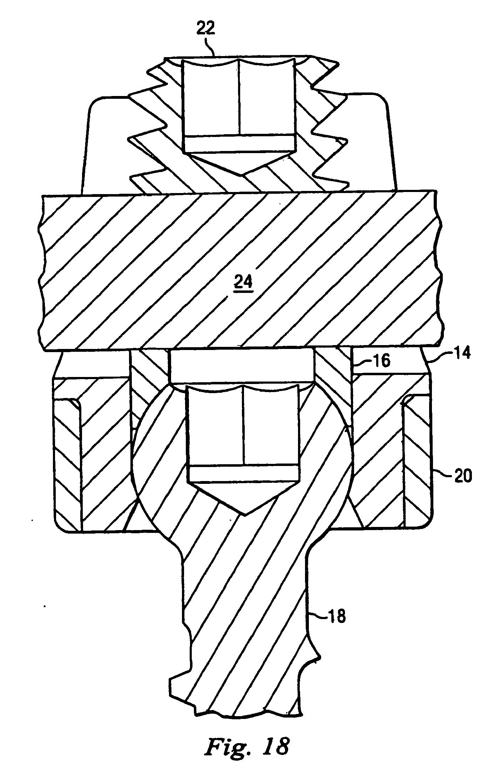

Fig. 18 is a diagrammatic front cross-sectional view of the bone anchor assembly, rod, and set screw of Fig. 17 in the locked position.
Fig. 19 is a diagrammatic bottom view of the bone anchor assembly, rod, and set screw of Figs. 17 and 18 in the locked position.
Fig. 20 is a diagrammatic perspective view of a system according to another embodiment of the present disclosure.
Fig. 21 is a diagrammatic bottom view of the system of Fig. 20
Fig. 22 is a diagrammatic perspective cross-section view of the system of Figs. 20 and 21.
Fig. 23 is a diagrammatic side exploded view of the system of Figs. 20, 21, and 22.
Fig. 24 is a diagrammatic side view of a saddle according to another embodiment of the present disclosure.
Fig. 25 is a diagrammatic top view of the saddle of Fig. 24.
Fig. 26 is a diagrammatic bottom view of the saddle of Figs. 24 and 25.
Fig. 27 is a diagrammatic cross-sectional view of the saddle of Figs. 24, 25, and 26 taken along section line 27-27.
Fig. 28 is a diagrammatic bottom view of a sleeve according to another embodiment of the present disclosure.
Fig. 29 is a diagrammatic cross-sectional view of the sleeve of Fig. 28 taken along section line 29-29.
Fig. 30 is a diagrammatic perspective view of a bone anchor assembly according to another embodiment of the present disclosure.
Fig. 31 is a diagrammatic perspective view of a sleeve member according to another embodiment of the present disclosure.
Fig. 32 is a diagrammatic perspective view of a bone anchor assembly according to another embodiment of the present disclosure
Fig. 33 is a diagrammatic side view of the bone anchor assembly of Fig. 32.
Fig. 34 is a diagrammatic exploded perspective view of a bone anchor assembly according to another embodiment of the present disclosure.
Fig. 35 is a diagrammatic side view of a saddle member according to another embodiment of the present disclosure.
Fig. 36 is a diagrammatic exploded perspective view of a bone anchor assembly according to another embodiment of the present disclosure.
Fig. 37 is a diagrammatic front cross-sectional view of the bone anchor assembly of Fig. 36.
Fig. 38 is a diagrammatic front cross-sectional view of a saddle member and a crown member according to one embodiment of the present disclosure.
Fig. 39 is a diagrammatic exploded perspective view of a bone anchor assembly according to another embodiment of the present disclosure.
Fig. 40 is a diagrammatic front cross-sectional view of the bone anchor assembly of Fig. 39.
Fig. 41 is a diagrammatic perspective cross-sectional view of the bone anchor assembly of Fig. 39.
Fig. 42 is a diagrammatic perspective view of a saddle member and a crown member according to one embodiment of the present disclosure.
Fig. 43 is a diagrammatic front cross-sectional view of the saddle member and the crown member of Fig. 42.
Fig. 44 is a diagrammatic perspective view of a saddle member and a crown member according to one embodiment of the present disclosure.

### DESCRIPTION OF THE PREFERRED EMBODIMENTS

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications in the described devices, instruments, methods, and any further application of the principles of the disclosure as described herein are contemplated as would normally occur to one skilled in the art to which the disclosure relates. In particular, it is fully contemplated that the features, components, and/or steps described with respect to one embodiment may be combined with the features, components, and/or steps described with respect to other embodiments of the present disclosure.

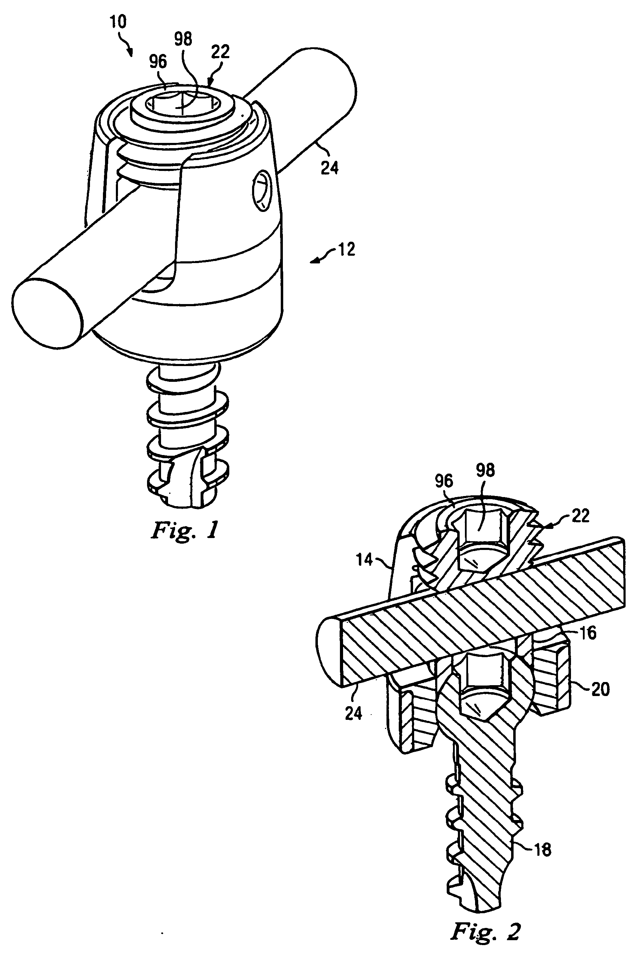

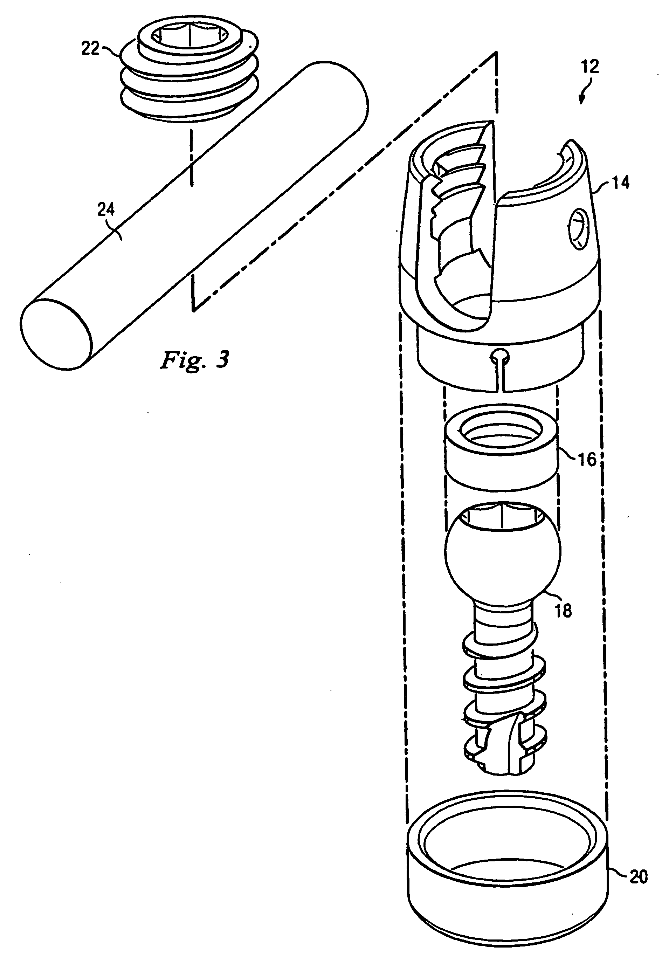

Referring to Figs. 1-3, a system 10 for orthopedic implantation is shown according to one embodiment of the present disclosure. The system 10 includes a bone anchor assembly 12. The bone anchor assembly 12 includes a receiver or saddle 14, a crown washer 16 (Figs. 2 and 3), a bone anchor 18, and a sleeve 20. A set screw 22 secures a rod 24 to the bone anchor assembly 12. The system 10 may be part of a larger orthopedic system comprising a plurality of longitudinal members (e.g., rods, plates, etc.), a plurality of bone anchor assemblies, and/or a plurality of connectors. In some embodiments, the system 10 is particularly suited for use in the spinal column. It will be understood that various types of fasteners or connectors (e.g. clamps) can be used in combination with the bone anchor assembly 12 and rod 24. Further additional or alternative longitudinal members can also be used, such as the plates and/or rods disclosed in U.S. Pat. No. 6,485,491.

The components of system 10 can be implanted via an open, minimally-invasive, or other surgical approach. Generally, one or more bone anchor assemblies 12 are inserted into one or more bones, then the longitudinal members 24 are contoured, if necessary, and surgically inserted and connected to the bone anchor assemblies. The relative angles of bone anchor assemblies 12 with respect to the longitudinal members 24 can be adjusted as necessary for ease of connection of the longitudinal member to the fasteners. Additional connectors are fitted to the longitudinal members and/or bone anchors as necessary or desired, and all elements are locked against unwanted movement with respect to other parts.

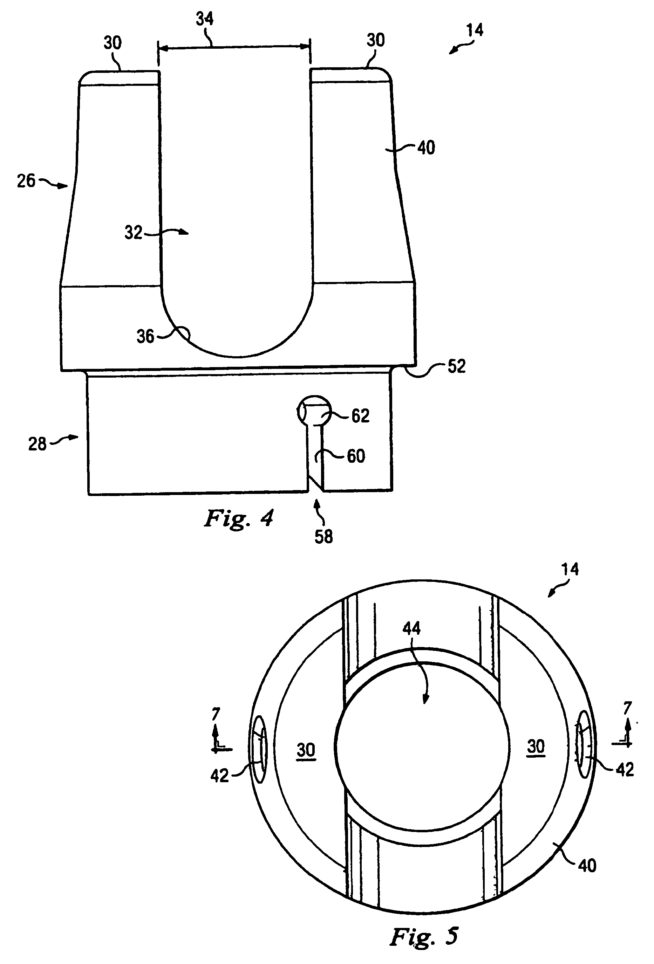

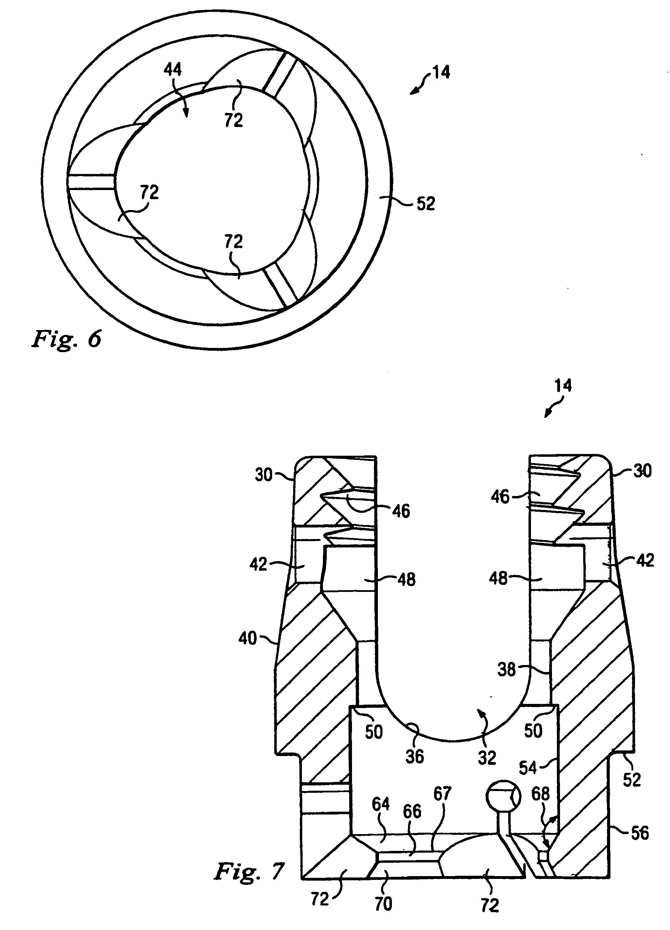

Referring now to Figs. 4-12, the components of the bone anchor assembly 12 are shown individually in greater detail. Referring more specifically to Figs. 4-7, the receiver member or saddle 14 is illustrated therein. Fig. 4 is a diagrammatic side view of the saddle 14; Fig. 5 is a diagrammatic top view of the saddle 14; Fig. 6 is a diagrammatic bottom view of the saddle 14; Fig. 7 is a diagrammatic cross-sectional view of the saddle 14.

Saddle member 14 generally has a U-shape with an upper portion 26 and a lower portion 28. The upper portion 26 comprises two upright portions 30 that define a channel 32 extending through saddle member 14. Channel 32 is configured to accommodate an elongated member, such as rod 24. It is understood that the elongated member may have a number of desired lengths and diameters. In that regard, the width 34 of the channel 32 in the current embodiment is substantially equal to the diameter of elongated member. In some embodiments, the width of the channel is slightly larger than the diameter of the elongated member, which allows easier insertion of elongated member into the channel 32, allows for contouring of the elongated member, and also allows a variety of elongated member of differing sizes to be used with saddle member 14. Generally, the elongated member 24 is positioned above the bottom portion 36 of the channel 32 when in a locked position. However, in some embodiments the elongated member 24 may be seated within the bottom portion 36 when in a locked position. Thus, the bottom portion 36 may be shaped or otherwise include features to ensure secure placement of the elongated member. In the current embodiment, the bottom portion 36 has a substantially cylindrical shape, as viewed in Fig. 4, to allow clearance of the outer surface of the rod 24.

The upright portions 30 of the saddle member 14 include an inner surface 38 and an outer surface 40. A bore or hole 42 extends through the upright portions 30 between the outer surface 40 and the inner surface 38. The holes 42 are substantially aligned with one another and are substantially perpendicular to the channel 32. In some instances, the holes 42 are utilized for grasping by a surgical tool to facilitate positioning of the rod 24 into the bone anchor assembly 12 within the patient. In the current embodiment and as shown in Figs. 4 and 7, the outer surfaces 40 taper with respect to inner surfaces 38 as they extend upwardly. This taper reduces the bulk and size of the saddle member 14 allowing for easier handling. In that regard, a surgical instrument may engage the holes 42 without substantially increasing the overall width needed to insert to the bone anchor assembly.

Generally, the inner surfaces 38 extend substantially coaxially with the axis of a bore 44 extending longitudinally through saddle member 14. The inner surfaces 38 of the upright portions 30 define an internally threaded portion 46, as shown in Fig. 7. Internally threaded portion 46 is configured to be threadingly coupled with set screw 22, as described below. The internally threaded portion 46 is configured such that the threads end above the rod 24 when the rod is secured within the saddle member 14. In some embodiments, as shown in Fig. 7, the inner surfaces 38 include an annular relief or cutout 48 that extends radially around bore 44 below the threaded portion 46. The annular relief 48 eliminates the helical thread run out often found on internal threads. In other embodiments, the inner surfaces do not include an annular relief 48. Further, in some embodiments the threaded portion 46 may not end above the rod 24 when the rod is secured within the saddle member 14. In further embodiments, the outer surfaces of the upright portions 30 define an externally threaded portion, instead of or in addition to the internally threaded portion 46. An externally threaded portion is configured to be threadingly coupled with a locking nut. In some embodiments with an externally threaded portion, at least the externally threaded portion of the outer surfaces 40 extend substantially parallel with the longitudinal bore 44 of the saddle member 14. Alternatively, saddle member 14 could be externally and/or internally configured for compression members using snapping, twisting or other types of closures.

The aperture or bore 44, which may be generally cylindrical, extends through the saddle member 14 from the upper portion 26 to the lower portion 28. The bore extends along the longitudinal axis of the saddle member 14 and substantially transversely to and in communication with channel 32. In the current embodiment, the bore 44 extends entirely through the saddle member 14. In other embodiments, the bore 44 extends only partially through the saddle member 14. As described below, the bore 44 can also allow access for a driving tool to engage the bone anchor 18. In this manner, the bone anchor 18 can be driven into a bone, such as a vertebra, with the bone anchor movably retained within the saddle member 14.

The inner surfaces 38 include a stop portion or shoulder 50 below the annular relief 48 and in communication with bore 44. The shoulder 50 is provided to act as a stop for the bottom-loaded crown member 16. Generally, shoulder 50 serves as an upper boundary for the crown member 16 within the bore 44, preventing movement of the crown beyond the shoulder. Though not shown in the current embodiment, in other embodiments the shoulder 50 and/or crown member 16 include features to facilitate engagement therebetween, such as mating projections and recesses.

The lower portion 28 of the saddle member 14 has a reduced diameter compared to the upper portion 26. Accordingly, a shoulder 52 is defined between the upper portion 26 and the lower portion 28. The lower portion 28 includes an inner surface 54 and an outer surface 56. The inner surface 54 at least partially defines bore 44. In that regard, the saddle member 14 is configured for bottom-loading. That is, the crown member 16 and the bone anchor 18 are inserted into the saddle member 14 through an opening in the bottom of the lower portion 28. In that regard the lower portion 28 must have an opening large enough to receive the crown member 16 and bone anchor 18. However, the opening cannot be too large as the crown member 16 and bone anchor must be retained within the saddle member 14. Accordingly, in the present embodiment the lower portion 28 includes slotted reliefs 58. The slotted reliefs 58 extend through the lower portion 28 from the inner surface 54 to the outer surface 56 and extend upwardly from the bottom of the lower portion. The slotted reliefs 58 include an elongated slot portion 60 and a relief 62 having an increased width compared to the slot portion. The slotted reliefs 58 render the lower portion 28 at least partially flexible so that the lower portion is movable between at least two positions; the first position being an enlarged insertion configuration for allowing the head of the bone anchor to pass therethrough; the second position being a reduced dimension retaining configuration for retaining the head of the bone anchor therein. The lower portion 28 may flex outwardly (expand) slightly to allow insertion of the bone anchor 18 and/or crown 16 into bore 44. The lower portion 28 may also flex inwardly (contract) to secure the bone anchor 18 and crown 16 within the bore 44. In that regard, as described below engagement with the sleeve 20 may cause the lower portion 28 to contract and thereby retain the bone anchor 18 and crown 16 therein. There are three slotted reliefs 58 in the current embodiment. The three slotted reliefs are equally spaced 120 degrees apart from one another around the circumference of the lower portion 28. Two of the slotted reliefs 58 are offset approximately 30 degrees from the axis of channel 32, while the other slotted relief 58 is offset approximately 90 degrees from the axis of the channel 32. In other embodiments, the number, shape, size, and placement of the slotted reliefs may be varied.

As described, the inner surface 54 is configured to receive the head of the bone anchor 18. In that regard, the inner surface 54 includes a tapered portion 64 extending inwardly from the inner surface to a cylindrical bore 66, thereby defining an edge 67. In some embodiments, the tapered portion 64 has a substantially conical shape. The bore 66 defines the narrowest opening of the bore 44 in the lower portion 28. The intersection of the tapered portion 64 and the cylindrical bore 66 generally define the edge 67 which comprises the seat of the saddle 14 configured for mating with the bone anchor 18. In that regard, the tapered portion 64 extends from the inner surface 54 at an angle 68. As illustrated in Fig. 7, the angle 68 may have a value between 90 and 170 degrees, which may also be considered 10-90 degrees relative to the longitudinal axis of the saddle 14. In one particular embodiment, the angle 68 is approximately 20 degrees. In another particular embodiment, the angle 68 is approximately 55 degrees. When assembled, the head of the bone anchor 18 engages the edge 67 such that at least some of the loading forces from the bone anchor are directed radially outward to the sleeve 20. Distributing the pullout force radially may increase the carrying load capacity of the bone anchor assembly 12 and reduces the risk of disassembly during rod reduction and/or postoperatively.

The inner surface 54 also includes a tapered portion 70 extending outwardly from the cylindrical bore 66. The tapered portion 70 is configured to allow the bone anchor 18 to translate through a plurality of positions corresponding to multi-axial movement with respect to the saddle 14 by reducing the interference of the lower portion 28 with the shaft of the bone anchor. In some embodiments, the tapered portion 70 has a substantially conical shape.

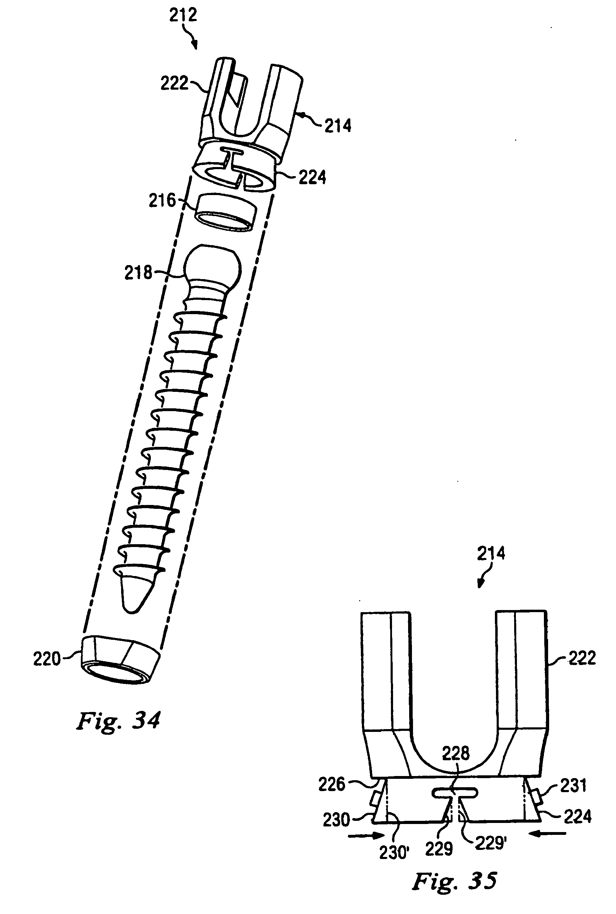

Generally, the outer surface 56 is substantially cylindrical and configured to mate with the sleeve 20. In the current embodiment, the outer surface 56 is substantially flat and coaxial with the longitudinal axis of the saddle 14 when in a neutral position (i.e., not expanded or contracted). In other embodiments, however, the outer surface may be flared outward (see e.g., Fig. 34) or inward when in a neutral position. In some embodiments the outer surface 56 and/or a portion of the sleeve 20 may be chamfered to facilitate engagement therebetween. In some embodiments, the neither the outer surface 56 nor the sleeve 20 is chamfered. Rather, the engaging edges of the outer surface 56 and the sleeve 20 comprise break edges. In some embodiments, the break edges are less than .5 mm. In one particular embodiment, the break edge of the outer surface 56 is less than .1 mm and the break edge of the sleeve 20 is less than .4 mm. Further, the outer surface 56 and/or the inner surface of the sleeve 20 may be treated (physically, chemically, and/or combinations thereof) to encourage engagement therebetween. The shoulder 52 serves as an upper stop for sleeve 20. In yet other embodiments, the outer surface 56 and/or the inner surface of the sleeve 20 may include mating features (e.g., projections and recesses) to secure engagement and/or alignment therebetween.

Engagement between the sleeve 20 and the outer surface 56 of the lower portion 28 results in the bone anchor 18 and crown 16 being movably held within the saddle member 14. That is, the bone anchor 18 and crown 16 are securely retained with the saddle member 14, but are capable of movement with respect to the saddle member. In particular, the bone anchor 18 is capable of multi-axial movement with respect to the saddle member 14. The bone anchor 18 locked with respect to the saddle 14 upon compression of the rod 24 by the set screw 22, which in turn compresses the crown member 16 onto the head of the bone anchor 18, thereby securing the bone anchor between the crown member and the seat of the saddle limiting movement of the bone anchor relative to the saddle.

As described above, the inner surface 54 includes the tapered portion 70 configured to allow the bone anchor 18 to translate through a plurality of positions corresponding to multi-axial movement with respect to the saddle 14 The inner surface 54 also includes angular cutouts 72 placed symmetrically about the circumference of the bore 44 to increase the allowable angulation of the bone anchor 18 in relation to the saddle 14. As shown in Fig. 6, there are three angular cutouts that are generally partially cylindrical in shape and at approximately a 45 degree angle with respect to the longitudinal axis of the saddle 14. The three angular cutouts 72 are equally spaced 120 degrees apart from one another around the circumference of the lower portion 28. Two of the angular cutouts 72 are offset approximately 30 degrees from the axis of channel 32, while the other cutout 72 is offset approximately 90 degrees from the axis of the channel 32. In the current embodiment, the slotted reliefs 58 are substantially aligned with and positioned within the cutouts 72. In other embodiments, the slotted reliefs 58 and cutouts 72 may be separated. Though a particular arrangement has been described above, it is understood that the number of cutouts, the shape of the cutouts, the position of the cutouts in relation to the axis of the channel 32, the angle of the cutouts in relation to the longitudinal axis of the saddle 14, the size of the cutouts, and the angular spacing between each cutout may vary for specific applications. Generally, the cutouts allow a greater range of motion between the saddle 14 and the bone anchor 18.

The illustrated embodiment of saddle member 14 is an "open" variety. That is, channel 32 is open through the top of saddle member 14, thereby making the saddle member 14 generally U-shaped. It will be understood that the principles of this disclosure apply to equally to "closed" fasteners, i.e., those in which a longitudinal member receiving channel is not open through the top, but is essentially a bore through the saddle member 14.

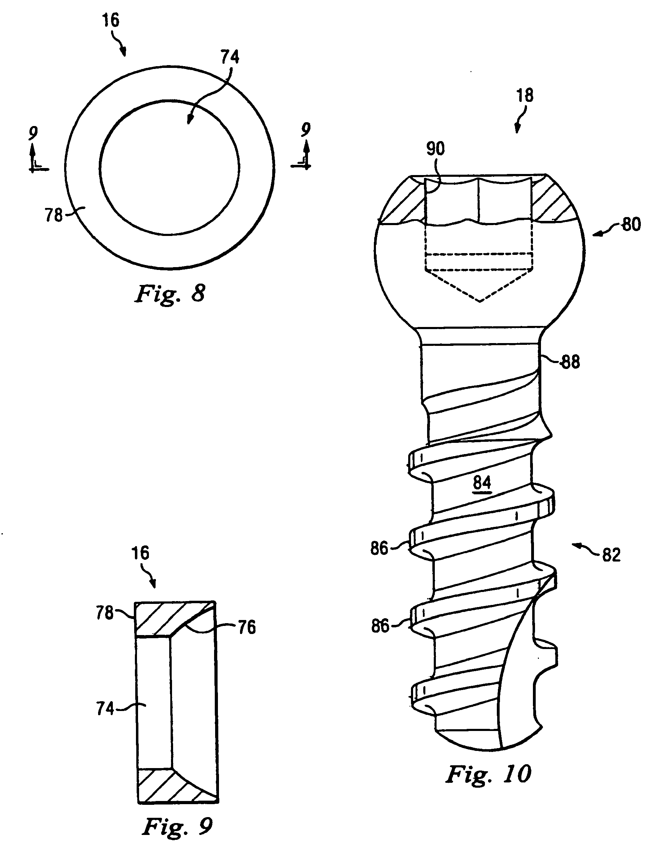

Referring more specifically to Figs. 8 and 9, the crown member or washer 16 is illustrated therein. Fig. 8 is a diagrammatic top view of the crown washer 16; Fig. 9 is a diagrammatic cross-sectional view of the crown washer 16.

The crown member 16 is substantially cylindrical with an internal opening 74 and an undersurface 76. Crown member 16 is sized to fit within bore 44 of the saddle, so that crown member 16 has some freedom of axial movement within the bore. In particular, crown member 16 is sized to move axially between shoulder 50 and the head of the bone anchor 18. Internal opening 74 in the crown member 16 allows surgical instrument access to the bone anchor 18 when the crown member is positioned above or on top of the bone anchor. Undersurface 76 is configured to accommodate at least a portion of the head of the bone anchor 18. The undersurface 76 may be shaped (e.g. spherical, rounded, conical, or otherwise) to allow relative multi-axial movement between the crown and the head of the bone anchor 18. In the current embodiment, the undersurface 76 is partially rounded or spherical to mate with the spherical head of the bone anchor. In that regard, the rounded portion of the undersurface 76 has substantially the same radius of curvature as the head of the bone anchor 18. The undersurface 76 is shaped such that sufficient compression of the head of the bone anchor 18 between the crown member 16 and the seat of the saddle 14 can fixedly secure the bone anchor relative to the saddle.

An upper surface 78 of the crown member 16 is configured to engage with the elongated member of spinal rod 24. In particular, the crown member 16 is configured to be compressed downwardly by the spinal rod 24 to secure the bone anchor in place. In some embodiments, the upper surface 78 includes features to facilitate engagement with the elongated member. For example, in at least one embodiment the upper surface 78 includes a recess shaped to match the outer contours of the elongated member. In other embodiments, the crown member 16 includes additional features to facilitate proper positioning of the crown member within the saddle 14, such as external grooves or projections configured to mate with corresponding projections or grooves of the saddle.

Referring more specifically to Fig. 10, the bone anchor 18 is illustrated therein. Fig. 10 is a diagrammatic side view of the bone anchor 18. As shown, the bone anchor 18 is a bone screw having a head portion 80 and a shaft portion 82. The shaft portion 82 includes a bone engaging portion 84 that, in the current embodiment, includes a series of threads 86. The threads 86 are particularly suited for engaging bone. The shaft portion 82 also includes a non-threaded shank portion 88. In the current embodiment, the head portion 80 of the bone anchor 18 includes a substantially spherical outer contour. However it should be understood that any external contour that allows for multi-axial movement could be utilized. In the current embodiment, the spherical surface 18 of the head portion 80 provides for bearing contact with the seat of the saddle 14 so that the bone screw can be arranged at a variety of angular orientations relative to the saddle corresponding to the multi-axial movement. In the illustrated embodiment, a tool-engaging recess 90 is formed in the upper portion of head portion 80. The specific shape of tool-engaging recess 90 may be chosen to cooperate with any suitable driving tool. In the current embodiment, the recess 90 is a hex-shaped recess configured to receive a hex-driver. In relation to each other, the maximum diameter of the bone-engaging portion 84 may be greater, smaller, or equal to the maximum diameter of the head portion 80, and at least a portion of the non-threaded portion 88 has a maximum diameter less than the maximum diameter of the head portion 80. The reduced diameter of the non-threaded portion 88 may increase the available range of motion of the bone anchor 18 when assembled within the saddle 14.

The size of the bone anchor 18 is selected based on the intended use. Generally, the bone anchor 18 may have a length between 10-52 mm. However, in some circumstances the bone anchor 18 may be larger or smaller than this range. It will be understood that other bone anchors may be utilized. For example, in some embodiments a bone anchor including a hook element is utilized. Such a hook includes a head portion identical or substantially similar to head portion 80 of bone anchoring member 18. However, the shank portion of such a bone anchor would include or extend into a curved portion for engaging with and/or connecting to a bone. It should be understood that while specific bone anchors have been described herein, bone anchors of various head design, shaft design, thread pitch, and/or tip taper suitable for orthopedic use may be utilized.

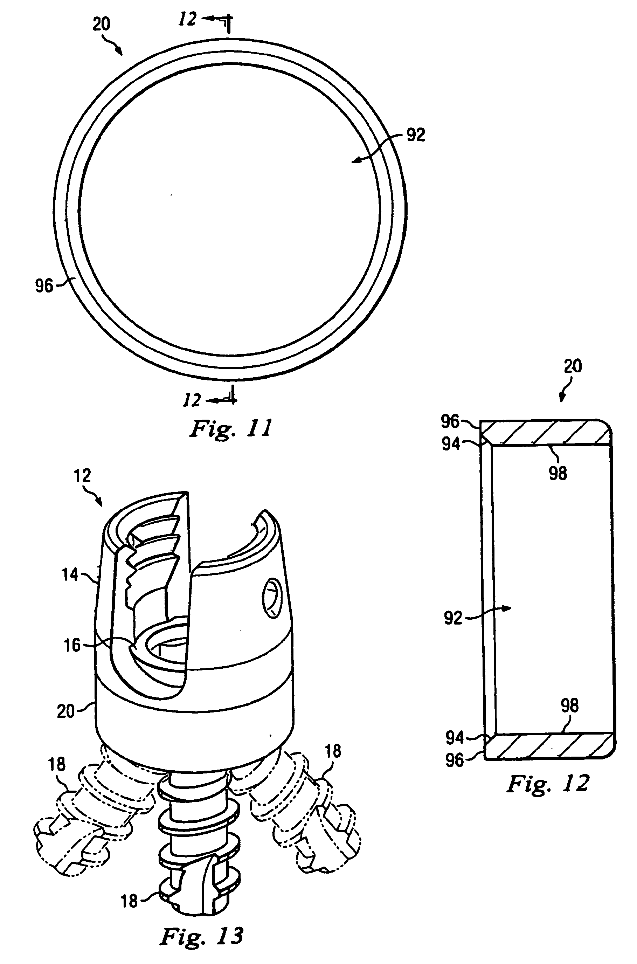

Referring more specifically to Figs. 11 and 12, the sleeve 20 is illustrated therein. Fig. 11 is a diagrammatic bottom view of the sleeve 20; Fig. 12 is a diagrammatic cross-sectional view of the sleeve 20.

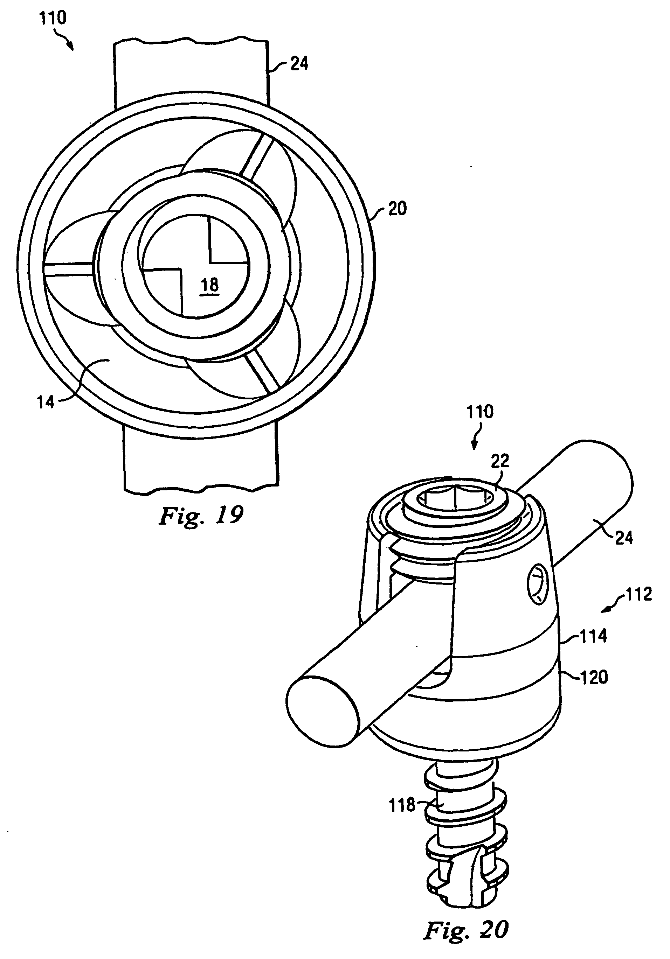

The sleeve 20 is substantially cylindrical with an opening 92 extending therethrough. The sleeve 20 is sized to friction fit around the lower portion 28 of the saddle 14. In particular, the sleeve 20 is sized to retain the displaceable lower portion 28 around the head portion 80 of the bone anchor 18 to secure the bone anchor therein. In that regard, the sleeve 20 prevents the lower portion 28 of the saddle 14 from flexing outwardly to ensure that the head of the bone anchor is retained therein. It is recognized that in some embodiments the sleeve 20 allows some outward flexing of the lower portion 28, but still retains the bone anchor securely therein. In some embodiments, the sleeve 20 compresses the lower portion 28 of the saddle 14 inwardly to retain the bone anchor. In the current embodiment the diameter of the opening 92 is slightly less than the diameter of the outer surface 56 of the lower portion 28 when in a neutral position. Thus, when the sleeve 20 is frictionally engaged with outer surface 56 the lower portion 28 is prevented from expanding outwardly. To facilitate the initial advancement of the sleeve 20 over the outer surface 56, the sleeve includes a chamfer 94 between an upper surface 96 and an inner surface 98. The chamfer 94 serves to guide the sleeve 20 around the outer surface 56. In other embodiments, the lower portion 28 of the saddle 14 may include a chamfer and the chamfer 94 of the sleeve may be omitted. To ensure that the sleeve 20 has sufficient strength to prevent outward flexing of the lower portion 28 of the saddle and also prevent the lower portion of the saddle from stretching the sleeve 20, the sleeve 20 may be formed of a material having an increased hardness with respect to the saddle 14 or at least the lower portion of the saddle. In addition to or in lieu of using a harder material for the sleeve, in some embodiments the thickness of the sleeve 20 may be increased and the thickness of the lower portion 28 decreased to increase the strength of the sleeve relative to the lower portion (e.g., see Figs. 20-24).

The upper surface 96 of the sleeve 20 is configured to engage with the shoulder 52 of the saddle 14. In the current embodiment the upper surface 96 is substantially smooth and mates with the substantially smooth surface of the shoulder 52. However, in other embodiments the upper surface 96 and/or the shoulder 52 may include mating features to secure the position of the sleeve 20 relative to the saddle 14. Similarly, in other embodiments the inner surface 98 and/or the outer surface 56 may include mating features to secure the position of the sleeve 20 relative to the saddle 14.

Referring again to Figs. 1-3, the set screw 22 is illustrated as an externally threaded element. The set screw 22 may be a standard set screw or a break-offset screw such as those disclosed in U.S. Pat. No. 6,478,795. The set screw may also include reverse angle threads as disclosed in U.S. Pat. No. 6,296,642. In the current embodiment, the set screw 22 is configured to thread into threaded portion 46 of the saddle 14. The set screw 22 advances along threaded portion 46 until it engages the rod 24 and urges the rod towards the bottom portion 36 of the channel 32, thereby compressing crown member 16 downward and locking the head portion 80 of the bone anchor 18. The set screw 22 includes an upper surface 96 having a tool-engaging recess 98. The set screw 22 is sized such that the upper surface 96 will be substantially aligned with or below the top of the saddle 14 after locking engagement to minimize the overall profile of the system 10. In other embodiments, the set screw 22 may extend beyond the top of the saddle 14 after locking engagement. The upper surface 96 may be rounded to reduce internal trauma to a patient or a substantially flat to minimize the profile. The tool-engaging recess 98 is configured to mate with a tool used for introducing the set screw 22 into saddle member 14. In the current embodiment, the recess 98 is a hex-shaped recess configured to receive a hex-driver. In some embodiments, the recess 98 is substantially similar to the recess 90 of the bone anchor 18 such that the same surgical driving instrument may be utilized with both the set screw 22 and the bone anchor. The specific shape of tool-engaging recess 98 may be chosen to cooperate with any suitable driving tool.

Alternatively or additionally, set screw 22 can comprise an external element such as a nut or cap. The external element which may or may not include threads or other features for holding the external element to receiver member 30. If an external element is used, the saddle 14 may be provided with compatible threads or other features for mating with the external element. Generally, set screws, locking screws, locking nuts, nuts, and combinations thereof used in this manner may be referred to herein as compression members.

Different elasticities and hardnesses depending on application. Cobalt-chrome, titanium, PEEK, et al.

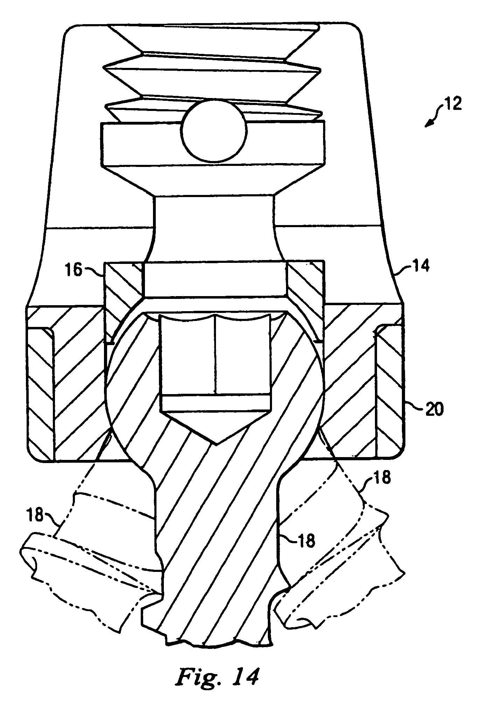

Referring now to Figs. 13 and 14, shown therein is the bone anchor assembly 12. Fig. 13 is a diagrammatic perspective view of the bone anchor assembly 12; Fig. 14 is a diagrammatic front cross-sectional view of the bone anchor assembly 12. In some embodiments, the bone anchor assembly 12 is assembled as follows. The crown member 16 is introduced into the bore 44 of the saddle 14 through the lower portion 28. In some embodiments, the crown member 16 is sized such that it has a diameter less than the diameter of the opening of the lower portion 28 of the saddle 14 such that the crown member 16 may be inserted into the bore 44 without expansion or flexing of the lower portion. In other embodiments, the crown member 16 is sized such that it has a diameter larger than the diameter of the opening of the lower portion 28 of the saddle 14 such that the lower portion is expanded about the slotted reliefs 58 as the crown member is introduced. Upward movement of the crown member 16 within the bore 44 is limited by shoulder 50.

Once the crown member 16 is positioned within the bore 44, the head portion 80 of the bone anchor 18 is introduced into the bore 44 of the saddle 14 through the lower portion 28. Introduction of the bone anchor 18 may require expansion of the lower portion 28 about the slotted reliefs 58. Once positioned within the bore 44, the upper section of the head portion 80 moveably engages the undersurface 76 of the crown member 16 and the lower section of the head portion moveably engages the seat of the saddle 14. After the crown member 16 and bone anchor 18 have been positioned within the bore 44, the sleeve 20 is positioned around the lower portion 28 until the upper surface 96 of the sleeve engages the shoulder 52 of the saddle 14. The sleeve 20 thereby holds the lower portion 28 around the head portion 80 of the bone anchor 18 securing the head portion therein. The sleeve 20 prevents outward movement or flexing of the lower portion 28 that results in the bone anchor 18 and crown 16 being movably held within the saddle member 14. That is, the bone anchor 18 and crown 16 are securely retained with the saddle member 14, but are capable of movement with respect to the saddle member. In particular, the bone anchor 18 is capable of multi-axial movement with respect to the saddle member 14 after the sleeve 20 has been introduced (e.g., see the ghost views of the bone anchor 18 in Figs. 13 and 14).

In some embodiments, the sleeve 20 and the saddle member 14 engage one another via a friction fit. In that regard, the diameter of the inner opening of the sleeve 20 may be substantially similar to the outer diameter of the lower portion of the saddle member 14. In some embodiments, the diameter of the inner opening of the sleeve 20 is less than the outer diameter of the lower portion of the saddle member. To encourage the friction fit, the outer surface 56 of the saddle 14 and/or the inner surface of the sleeve 98 may be roughened, textured, knurled, grit-blasted, or otherwise treated. In that regard, it is contemplated that the surfaces may be physically or chemically etched. Further, it is also contemplated that a biocompatible adhesive be utilized to further secure the sleeve 20 and the saddle member 14. In other embodiments, the sleeve 20 and/or the saddle 14 may include mating features (e.g., projections and recesses) to secure engagement and/or alignment therebetween. Once the sleeve 20 is securely attached to the saddle member 14, via friction fit or otherwise, the bone anchor assembly 12 is assembled. After assembly, the bone anchor assembly 12 may be provided to a surgeon for use in a surgical procedure. In many instances a plurality of bone anchor assemblies 12 will be provided as part of a surgical kit.

In use, the bone anchor assembly 12 may be implanted with the rod 24 and the set screw 22 as part of a orthopedic system as follows. One or more surgical exposures are made proximate to an area of the spine or other bones to be instrumented. The surgical exposures may be open, minimally-invasive, or of other types that are known in surgical practice. The vertebrae or other surgical site is prepared, for example by retracting tissue, removing tissue, drilling pilot holes, adjusting bony or other tissue, and/or other steps to prepare and fixate a bone or bones.

Prior to insertion of the bone anchor 18, the saddle 14, crown member 16, bone anchor 18, and sleeve 20 are assembled as described and shown above. In this pre-insertion state, the saddle 14 is multi-axially positionable and rotatable with respect to bone anchor 18, so that the channel 32 can be oriented in a plurality of positions with respect to the bone anchor. The surgeon is able to change the relative orientation of saddle 14 with respect to bone anchor 18 immediately prior to and during surgery as desired by rotating the saddle about the head portion 80 of the bone anchor or vice-versa. Crown assembly 16 is held within the saddle 14 between the head portion 80 of the bone anchor 18 and shoulder 50. It will be appreciated that assembly of these parts can take place at any time prior to insertion, by the surgeon, manufacture, or otherwise, and that kits including one or more of each type of part described above, in one or more sizes can be provided for the surgeon's convenience.

Once the surgical site is prepared, the assembled bone anchor assembly 12 is implanted into the site. In the embodiment in which bone anchor 18 is a bone screw, the bone engaging portion 84 of the shaft 82 is inserted into a bone. In some embodiments, the bone is a vertebra or part thereof, such as a pedicle. In that regard, the vertebra or other bone may be prepared to receive the bone anchor 18. In some embodiments, the pilot hole or bore is predrilled or tapped into the vertebra for receiving the bone screw prior to the bone screw being inserted. In some embodiments, the bone screw is a self-drilling or self-tapping screw, and predrilling an opening may be omitted. An appropriate surgical tool or driver is engaged with the tool-engaging recess 90 of the bone anchor 18. The surgical driver is inserted through the upper portion 26 of the saddle 14 along bore 44, through the opening 74 in the crown member 16 to engage the bone anchor 18. The surgical driver is then utilized to rotationally insert the bone anchor, and thereby the bone anchor assembly 12, into the bone or vertebra. Preferably, the bone anchor 18 is driven into the vertebra to a recommended depth for adequate fixation, but preferably not so deep that the bottom of the saddle 14 will contact or press against the vertebral bone limiting the available movement thereof. In order for the multi-axial capability of the bone anchor assembly 12 to be utilized, the saddle 14 must be allowed to pivot in three dimensions about the head portion 80 of the bone anchor 18.

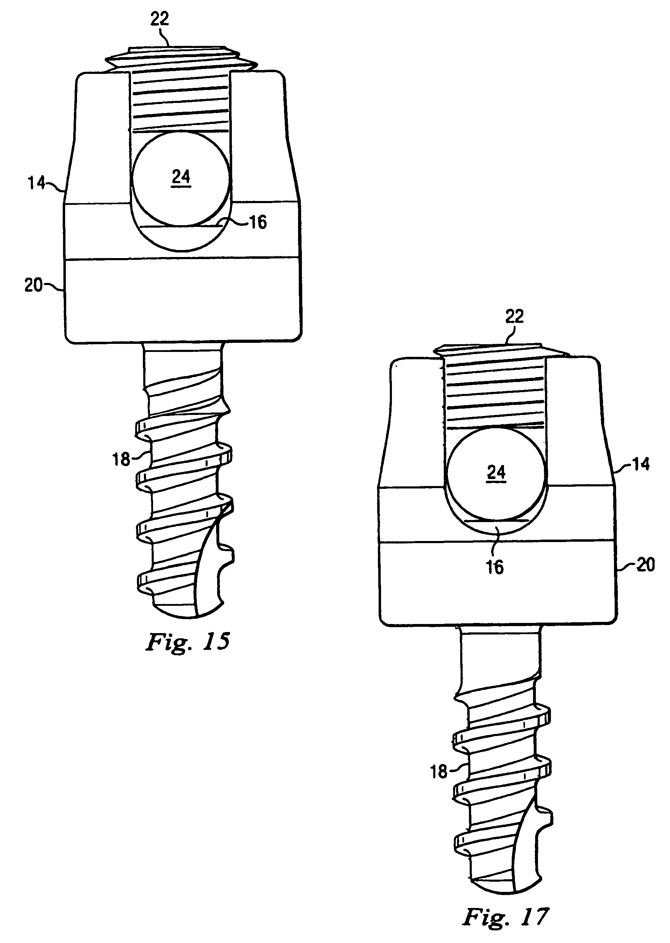

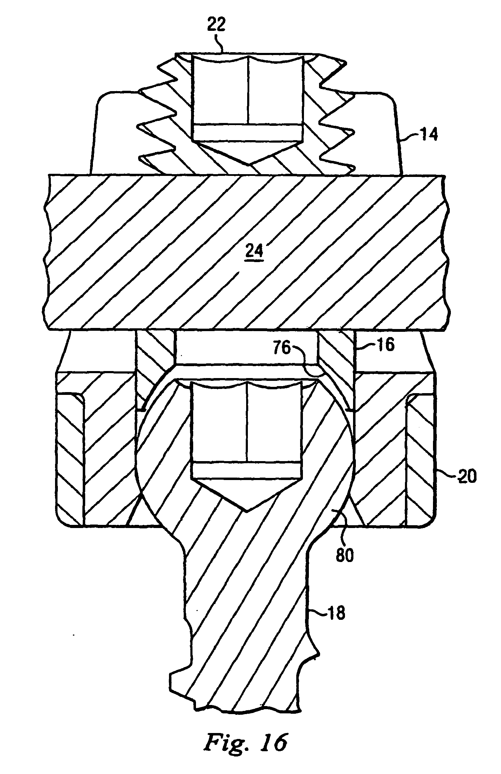

After the bone anchor 18 has been inserted into the bone to the desired depth, the surgical tool is removed, and the surgeon makes any desired adjustments to the orientation of saddle 14 with respect to bone anchor 18. For example, the surgeon may rotate or angle the saddle 14 relative to the bone anchor 18 to achieve a desired orientation. In particular, the surgeon may adjust the position of the saddle 14 to accommodate the reception of a longitudinal member, such as rod 24. A longitudinal member, such as rod 24, can be bent or otherwise contoured and then inserted into the surgical site. In particular, the rod 24 is inserted into channel 32 of the saddle. The rod 24 is inserted into towards the bottom portion 36 of the channel 32 at least to a point so that set screw 22 can be threadingly engage the threaded portion 46 of the saddle 14 to hold the rod 24 within the channel, as shown in Figs. 15 and 16.

As shown in Figs. 15 and 16, the rod 24 engages the upper surface 78 of the crown member 16. As shown, the crown member 16 extends at least partially above the bottom of the channel 32. In this position, the bone anchor 18 is still capable of multi-axial movement as illustrated by the space between the head portion 80 of the bone anchor 18 and the undersurface 76 of the crown member 16. It is recognized that there may not actually be a space between the undersurface 76 and the head portion 80. However, the space is shown to clearly illustrate that the head portion 80 is not locked in place in this orientation. Further, the lower section of the head portion 80 of the bone anchor 18 engages the seat portion of the saddle 14. This engagement between the head portion 18 and the seat portion directs the at least some of the loading forces from the bone anchor 18 outwardly toward the sleeve 20. Accordingly, at least some of the pullout forces may be distributed to the sleeve 20. Distributing the pullout forces radially increases the load to the sleeve 20 and reduces the risk of disassembly during rod reduction and/or postoperatively. Other types of longitudinal members may be used instead of rod 24. In that regard, the other longitudinal members may similarly be positioned within the channel 32 of the saddle 14. As the rod 24 and saddle 14 are still moveable with respect to the bone anchor 18, the surgeon can manipulate the spine and the implanted devices so that the spine is corrected or placed in a therapeutically improved position.

When the spine and implants are positioned as the surgeon desires, the rod 24 is locked within the channel 32 of the saddle 14 by advancing the set screw 22 against the rod. As the set screw 22 is advanced it urges the rod 24 towards the bottom portion 36 of the channel 32. The rod 24, in turn, presses against crown member 16 urging the crown member towards the head portion 80 of the bone anchor 18. The rod 24 is advanced until the head portion 80 is locked in place between the crown member 16 and the seat of the saddle 14. Generally, the rod 24 is positioned at least slightly above the bottom portion 36 of the channel 32 when in a locked position. The result is that the rod 24, saddle 14, and bone anchor 18 are locked in position with respect to one another, as shown in Fig. 17. As shown, in the locked position the top surface 96 of the set screw 22 is positioned substantially equal to or below the uppermost portion of the saddle 14, the rod 24 is seated within the bottom portion of the channel 32, and the undersurface 76 of the crown member 16 is engaged about the head portion 80 of the bone anchor. It should be noted that in some embodiments, the saddle 14, the head portion 80, and/or the rod 24 may be configured such that crown member 16 may be omitted. For example, in some embodiments the rod 24 may directly contact the head portion 80 to lock the relative positions of the bone anchor, saddle, and longitudinal member. Other bone anchor assemblies 12 and/or other implant devices are similarly tightened to hold the rod 24 and/or other members in the desired position.

As a part of the process of adjusting the position of the spinal column, one or more intervertebral implants may be inserted between adjacent vertebrae. Examples of such devices are disclosed in U.S. Pat. Nos. 5,984,967 and 6,113,637. "Cage"-type intervertebral implants may also be packed or otherwise provided with one or more substances for inducing or promoting bone growth, as disclosed in U.S. Pat. No. 5,984,967. Also, it is understood that the bone anchor assembly 12 and the associated devices may be implanted anteriorly, posteriorly, laterally, obliquely, combinations thereof, and/or in any other appropriate or necessary approach. Also, the bone anchor assembly 12 and associated devices may be used as a supplement to a Smith-Robinson technique.

In some aspects, the bone anchor assembly 12 as described above may be utilized to simplify a surgical procedure. For example, the bone anchor assembly 12 is threaded onto the vertebra from the top and the spinal rod 24 is loaded from the top, allowing a common surgical implantation approach. Also, the rod 24 does not need to be preloaded into the bone anchor assembly 12. Rather, the rod 24 can be loaded after implantation of the bone anchor assembly 12. Moreover, the multi-axial capability of the bone anchor assembly 12 allows the rod 24 to be connected with minimal contouring of the rod.

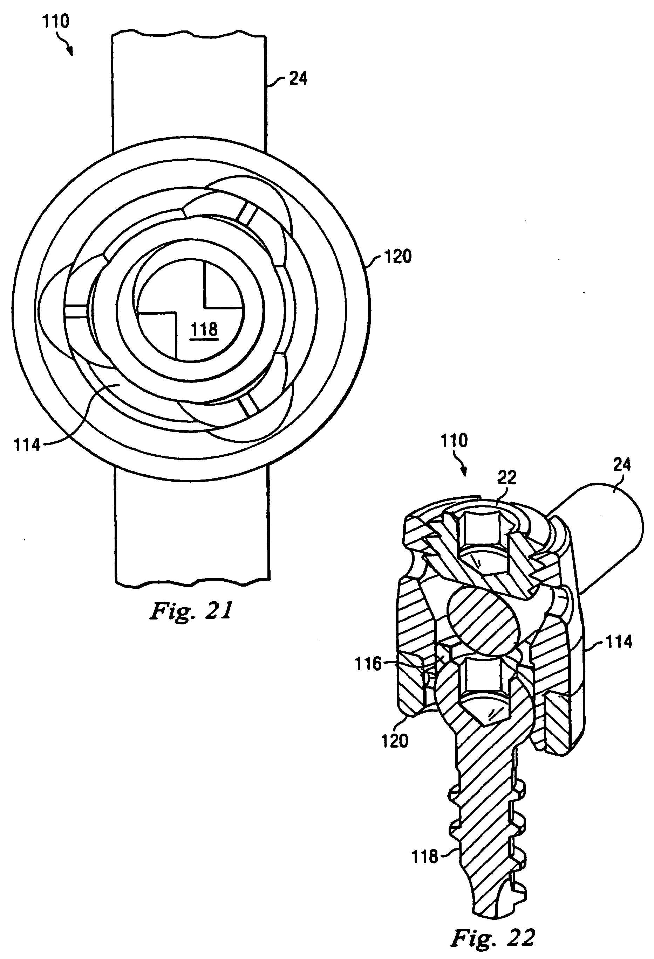

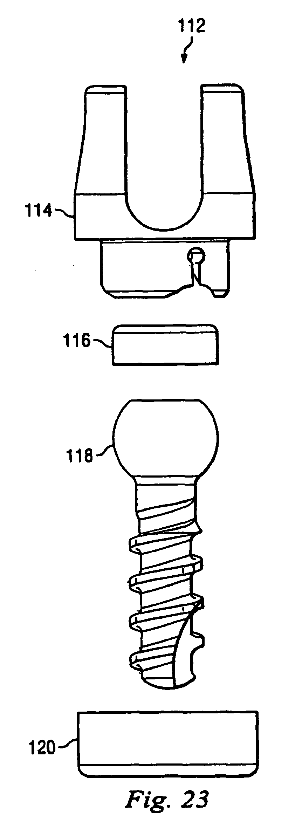

Referring to Figs. 20-23, a system 110 for orthopedic implantation is shown according to another embodiment of the present disclosure. The system 110 includes a bone anchor assembly 112. The bone anchor assembly 112 includes a receiver or saddle 114, a crown washer 116 (Figs. 22 and 23), a bone anchor 118, and a sleeve 120. A set screw 22 secures a rod 24 to the bone anchor assembly 112. In some aspects the system 110 is similar to the system 10 described above and may be assembled and utilized in substantially similar manners and therefore these aspects will not be described in detail. However, the sleeve 120 of system 110 has a larger thickness than the sleeve 20 of the bone anchor assembly 12 and the lower portion of the saddle 114 has smaller thickness than the lower portion 28 of the saddle 14. In some embodiments, the increased thickness of the sleeve 120 relative to the lower portion of the saddle 114 allows more of the pullout force from the bone anchor 118 to be transferred to the sleeve. Additional features of the bone anchor assembly 112 will now be described.

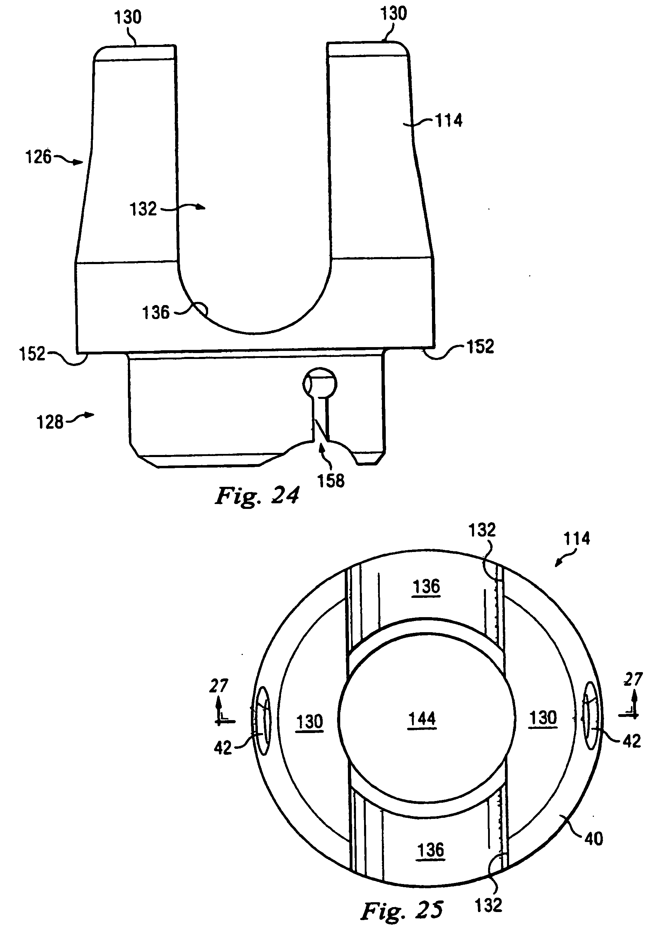

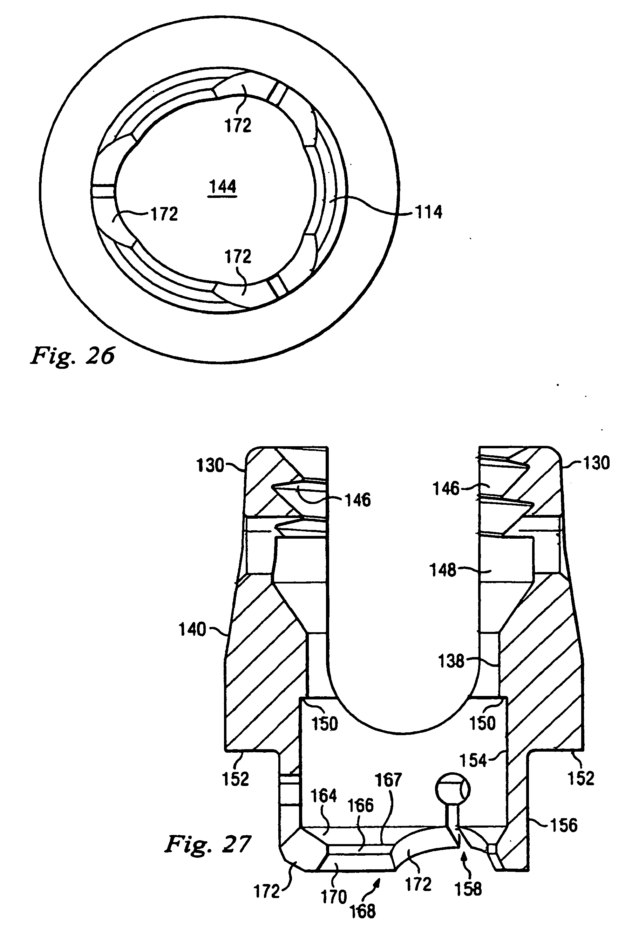

Referring now to Figs. 24-29, components of the bone anchor assembly 112 and, in particular, the saddle 114 and the sleeve 120 are shown individually in greater detail. Referring more specifically to Figs. 24-27, the receiver member or saddle 114 is illustrated therein. Fig. 24 is a diagrammatic side view of the saddle 114; Fig. 25 is a diagrammatic top view of the saddle 114; Fig. 26 is a diagrammatic bottom view of the saddle 114; Fig. 27 is a diagrammatic cross-sectional view of the saddle 114.

Saddle member 114 generally has a U-shape with an upper portion 126 and a lower portion 128. The upper portion 126 comprises two upright portions 130 that define a channel 132 extending through saddle member 114. Channel 132 is configured to accommodate an elongated member, such as rod 24. Generally, the elongated member 24 is positioned above a bottom portion 136 of the channel 132 when in a locked position. The upright portions 130 of the saddle member 114 include an inner surface 138 and an outer surface 140. A bore or hole 142 extends through the upright portions 130 between the outer surface 140 and the inner surface 138. The holes 142 are substantially aligned with one another and are substantially perpendicular to the channel 132. In the current embodiment and as shown in Figs. 24 and 27, the outer surfaces 140 taper with respect to inner surfaces 138 as they extend upwardly.

The inner surfaces 138 generally extend substantially coaxially with the axis of a bore 144 extending longitudinally through saddle member 114. The inner surfaces 138 of the upright portions 130 define an internally threaded portion 146, as shown in Fig. 27. Internally threaded portion 146 is configured to be threadingly coupled with set screw 22. The internally threaded portion 146 is configured such that the threads end above the rod 24 when the rod is secured within the saddle member 114. In the current embodiment and as shown in Fig. 27, the inner surfaces 138 include an annular relief or cutout 148 that extends radially around bore 144 below the threaded portion 146.

The aperture or bore 144, which may be generally cylindrical, extends through the saddle member 114 from the upper portion 126 to the lower portion 128. The bore 144 extends along a longitudinal axis of the saddle member 114 and substantially transversely to and in communication with channel 132. In the current embodiment, the bore 144 extends entirely through the saddle member 114. The bore 144 is configured to allow a driving tool access to the bone anchor 118. By engaging the bone anchor 118 through the bore 144 and driving the bone anchor into a bone, such as a vertebra, the bone anchor assembly 112 can be secured to the bone with the bone anchor movably retained within the saddle member 114. The inner surfaces 138 also include a stop portion or shoulder 150 below the annular relief 148 and in communication with bore 144. The shoulder 150 acts as a stop for the crown member 116.

The lower portion 128 of the saddle member 114 has a reduced diameter compared to the upper portion 126. A shoulder 152 is defined between the upper portion 126 and the lower portion 128 by the change in diameter. The lower portion 128 includes an inner surface 154 and an outer surface 156. The inner surface 154 at least partially defines bore 144. The lower portion 128 of the saddle member 114 is configured for bottom-loading. In that regard, the crown member 116 and the bone anchor 118 may be inserted into the saddle member 114 through an opening in the bottom of the lower portion 128. The bottom of the lower portion 128 includes opening large enough to receive the crown member 116 and bone anchor 118. However, the opening is not so large that the crown member 116 and bone anchor 118 cannot be retained within the saddle member 114.

In the present embodiment the lower portion 128 includes slotted reliefs 158. The slotted reliefs 158 extend through the lower portion 128 from the inner surface 154 to the outer surface 156 and extend upwardly from the bottom of the lower portion. The slotted reliefs 158 include an elongated slot portion 160 and a relief 162 having an increased width compared to the slot portion. In the current embodiment, the relief 162 is positioned at the uppermost end of the elongated slot portion. The slotted reliefs 158 render the lower portion 128 at least partially resiliently displacable or flexible. Accordingly, the lower portion 128 may flex outwardly (expand) slightly to allow insertion of the bone anchor 118 and/or crown 116 into bore 144. It is contemplated, however, that the opening in the bottom of the lower portion 128 be sized such that the head of the bone anchor 118 may be inserted without expansion of the lower portion. The lower portion 128 may also flex inwardly (contract) to secure the bone anchor 118 and crown 116 within the bore 144. In that regard, engagement with the sleeve 120 may cause the lower portion 128 to contract and thereby retain the bone anchor 118 and crown 116 therein. In some embodiments, the lower portion 128 may retain the bone anchor 118 and crown 116 therein in a substantially neutral position, that is, neither expanded nor contracted. There are three slotted reliefs 158 in the current embodiment. The three slotted reliefs 158 are equally spaced 120 degrees apart from one another around the circumference of the lower portion 128.

As mentioned above, the inner surface 154 is configured to receive the head of the bone anchor 118. In that regard, the inner surface 154 includes a tapered portion 164 extending inwardly from the inner surface to a cylindrical bore 166, thereby defining an edge 167. In some embodiments, the tapered portion 164 has a substantially conical shape. The bore 166 defines the narrowest opening of the bore 144 in the lower portion 128. The intersection of the tapered portion 164 and the cylindrical bore 166 generally define an edge 167, which comprises a seat 168 of the saddle 114 configured for mating with the bone anchor 118. In that regard, the tapered portion 164 may extend from the inner surface 154 at an angle between 90 and 170 degrees, which may also be considered 10-90 degrees relative to a longitudinal axis of the saddle 114. In the current embodiment, the angle is approximately 20 degrees. In another particular embodiment, the angle is approximately 55 degrees. When assembled the head of the bone anchor 118 engages the edge 167 such that at least some of the loading forces from the bone anchor are directed radially outward to the sleeve 120. Distributing the pullout force radially increases the load on the sleeve 120 and reduces the risk of disassembly.

The inner surface 154 also includes a tapered portion 170 extending outwardly from the bore 166. The tapered portion 170 is configured to allow the bone anchor 118 to translate through a plurality of positions corresponding to multi-axial movement with respect to the saddle 114 by reducing the interference of the lower portion 128 with the shaft of the bone anchor. In some embodiments, the tapered portion 170 has a substantially conical shape. The inner surface 154 also includes angular cutout portions 172 to increase the allowable angulation of the bone anchor 118 in relation to the saddle 114. As shown in Fig. 26, there are three angular cutout portions 172 that are generally partially cylindrical in shape and extend at approximately a 45 degree angle with respect to the longitudinal axis of the saddle 114. In the current embodiment, the three angular cutout portions 172 are equally spaced 120 degrees apart from one another around the circumference of the lower portion 128. In the current embodiment, the slotted reliefs 158 are substantially aligned with and positioned within the cutout portions 172.

The outer surface 156 of the lower portion 128 of the saddle 114 has a substantially cylindrical profile and is configured to mate with the sleeve 120. In the current embodiment, the outer surface 156 is substantially flat and coaxial with the longitudinal axis of the saddle 114 when in a neutral position (i.e., not expanded or contracted). Engagement between the sleeve 120 and the outer surface 156 of the lower portion 128 results in the bone anchor 118 and crown 116 being movably held within the saddle member 114. That is, the bone anchor 118 and crown 116 are securely retained within the saddle member 114, but are capable of movement with respect to the saddle member. In particular, the bone anchor 118 is capable of multi-axial movement with respect to the saddle member 114. The bone anchor 118 is locked with respect to the saddle 14 upon compression of the rod 24 by the set screw 22, which in turn compresses the crown member 116 onto the head of the bone anchor 118, thereby securing the bone anchor between the crown member and the seat 168 of the saddle movement of the bone anchor relative to the saddle.

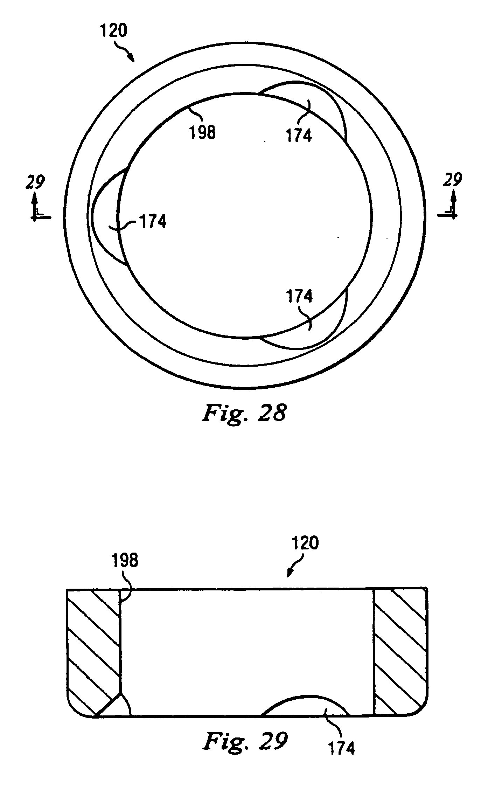

In the current embodiment, the sleeve 120 has a thickness greater than the thickness of the lower portion 128 of the saddle 114. In that regard, it is contemplated that the ratio of thicknesses between the sleeve 120 and the lower portion 128 of the saddle be in the range of 1.25:1 and 10:1. In some embodiments, the ratio of the thicknesses is approximately 2:1. Referring now to Figs. 26, 28, and 29, the sleeve 120 is shown therein. Fig. 28 is a diagrammatic bottom view of the sleeve 120; Fig. 29 is a diagrammatic cross-sectional view of the sleeve 120.

In the current embodiment the sleeve 120 includes angular cutout portions 174. The angular cutout portions 174 are configured to be substantially aligned with the angular cutout portions 172 of the lower portion 128 of the saddle 114 to increase the allowable angulation of the bone anchor 118 in relation to the saddle 114. Accordingly, there are three angular cutout portions 174 that are generally partially cylindrical in shape and extend at approximately a 45 degree angle with respect to the longitudinal axis of the sleeve 120. The three angular cutout portions 174 are equally spaced 120 degrees apart from one another around the circumference of the sleeve 120. The angular cutout portions 174 may have slightly increased width as compared to a continuation of angular cutout portions 172 to allow the cutout portions 172, 174 to be sufficiently aligned even when not perfectly aligned. In that regard, the angular cutout portions 174 may be slightly offset with respect to the angular cutout portions 172, yet the overall function of the cutouts is not adversely affected. In some embodiments, the outer surface 156 and/or the inner surface 198 of the sleeve 120 include mating features (e.g., projections and recesses) to facilitate alignment of the cutout portions 172 and 174 and/or engagement between the sleeve and saddle 114.

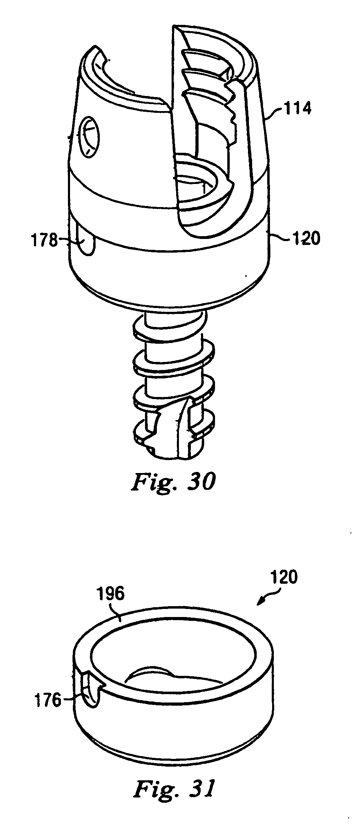

For example, referring now to Figs. 30 and 31, the sleeve 120 may include a recess 176 configured to receive a projection 178 of the saddle 114. As shown, the projection 178 may extend from the shoulder 152 of the saddle 114 and the recess 176 may be positioned adjacent an upper portion 196 of the sleeve 120 such that as the sleeve is urged over the lower portion 128 the projection engages the recess. If the projection 178 and recess 176 do not engage as the sleeve 120 is advance upwardly around the lower portion 128, then translation of the sleeve will be limited by engagement of the projection with an upper surface of the sleeve. The recess 176 and projection 178 are positioned such that alignment of the recess and projection corresponds with alignment of the angular cutout portions 172 and 174. It is understood that numerous other combinations of structures may be utilized to align the sleeve 120 with the saddle 114. Also, it is understood that the position of the structures may be varied. For example, it is contemplated that the outer surface 156 of the saddle 114 may include features for mating with corresponding features of the inner surface of the sleeve 120.

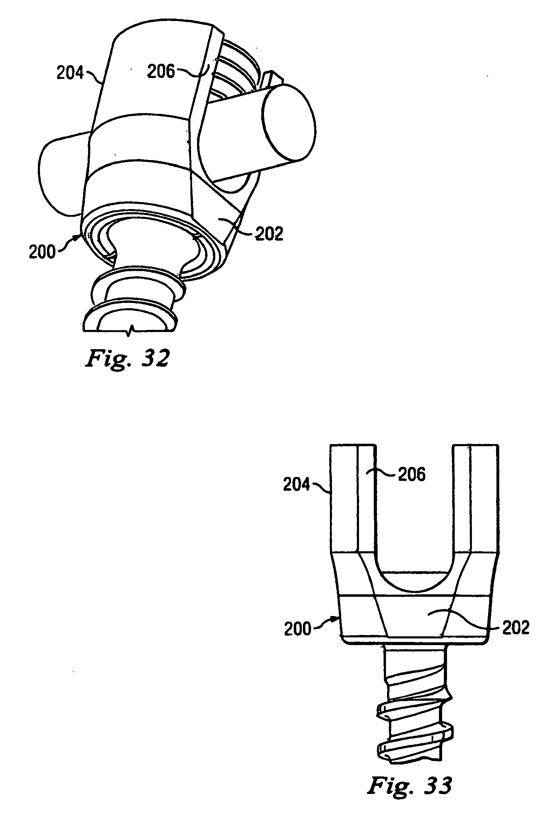

In some embodiments, the sleeve 20, 120 is not substantially cylindrical. Rather, in some embodiments at least one side of the sleeve is substantially planar. In some embodiments, two opposing outer portions of the sleeve have substantially planar outer profiles and two other opposing outer portions of the sleeve have substantially cylindrical outer profiles. For example, referring to Figs. 32 and 33, a sleeve 200 having planar surface 202 may be configured for use with a saddle 204 having planar outer surfaces 206 such that the planar surfaces of the sleeve and saddle are substantially aligned upon engagement between the sleeve and saddle. The planar surfaces 202, 206 of the sleeve and saddle may serve to limit the overall size and profile of the bone anchor assembly. An example of a saddle member having planar outer surface portions is disclosed in U.S. Patent No. 5,728,098.

Referring now to Figs. 34 and 35, shown therein is a bone anchor assembly 212 according to another embodiment of the present disclosure. Fig. 34 is an exploded perspective view of the bone anchor assembly; Fig. 35 is a side view of a saddle 214 of the bone anchor assembly. The bone anchor assembly 212 includes the receiver or saddle 214, a crown washer 216, a bone anchor 218, and a sleeve 220. Referring more specifically to Fig. 35, the saddle 214 may be substantially similar to the saddles 14 and 114 in some aspects. The saddle 214 includes an upper portion 222 and a lower portion 224. The lower portion 224 of the saddle member 214 has a reduced diameter compared to the upper portion 222 such that a shoulder 226 is defined between the upper and lower portions. The lower portion 224 is configured for bottom-loading. That is, the crown member 216 and the bone anchor 218 may be inserted into the saddle member 214 through an opening in the bottom of the lower portion 224. In that regard, the lower portion 224 must have an opening large enough to receive the crown member 216 and bone anchor 218. However, the opening cannot be so large as to prevent the crown member 216 and bone anchor from being retained within the saddle member 214. Accordingly, in the present embodiment the lower portion 224 includes slotted reliefs 228. The slotted reliefs 228 extend through the lower portion 224 and extend upwardly from the bottom of the lower portion. The slotted reliefs 228 include an elongated slot portion and a relief having an increased width compared to the slot portion. In the current embodiment, the elongated slot portion includes surfaces 229 that extend at an oblique angle with respect to a longitudinal axis of the saddle 214. In some embodiments, the angle of the surface 229 substantially matches the angle of an outer surface 230, as shown in Fig. 35. The slotted reliefs 228 render the lower portion 28 at least partially flexible or resiliently deformable.

In the current embodiment, the lower portion 224 is movable between a first position for receiving the crown and the head of the bone anchor and a second position for retaining the crown and the head of the bone anchor therein. In the first position, the lower portion 224 is flared outwardly with respect to a longitudinal axis of the saddle 214 such that an outer surface 230 of the lower portion extends at an oblique angle with respect to the longitudinal axis. In the second position, the lower portion 224 is contracted by the sleeve 220 to retain the crown 216 and bone anchor 218 therein. In that regard, a tool 231 may be utilized to initially contract the lower portion 224, as indicated by the arrows of Fig. 35, and then the sleeve 220 may be positioned around the lower portion to retain it in the second, contracted position. In Fig. 35, the dashed lines 229' and 230' illustrate the positions of the surfaces 229 and 230, respectively, when in the second position. In some embodiments, the outer surface 230 of the lower portion 224 extends substantially coaxially with the longitudinal axis of the saddle in the second position. In some embodiments, the outer surface 230 extends at an oblique angle with respect to the longitudinal axis in the second position.

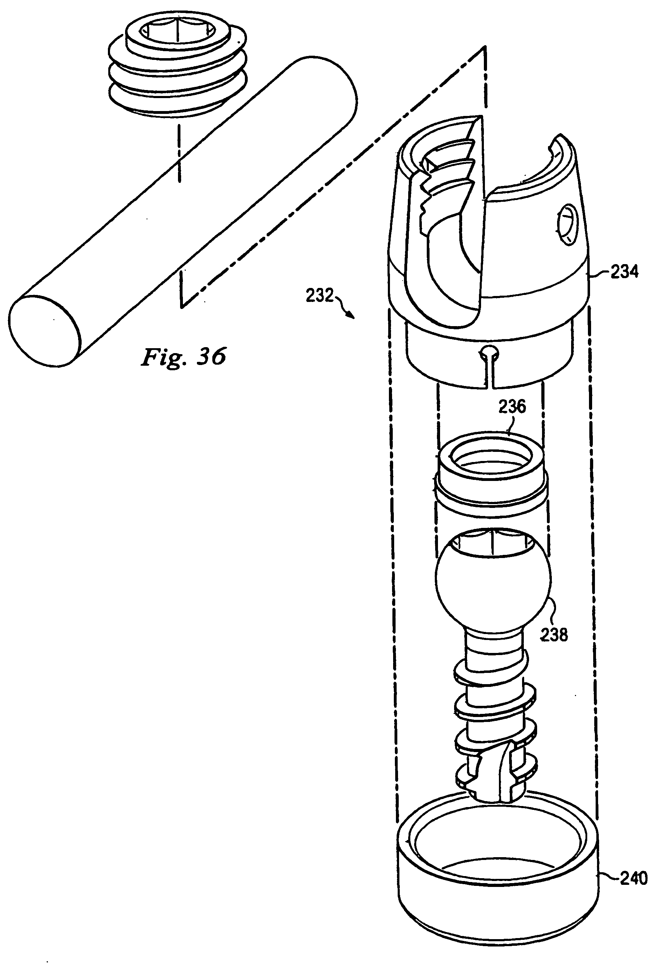

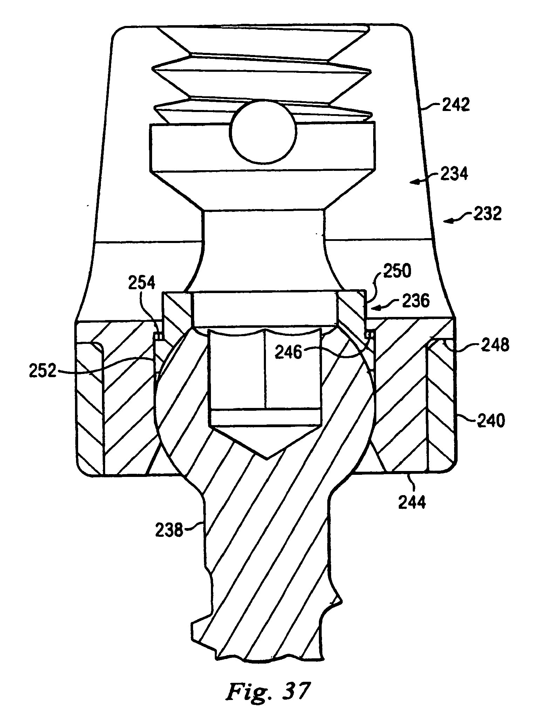

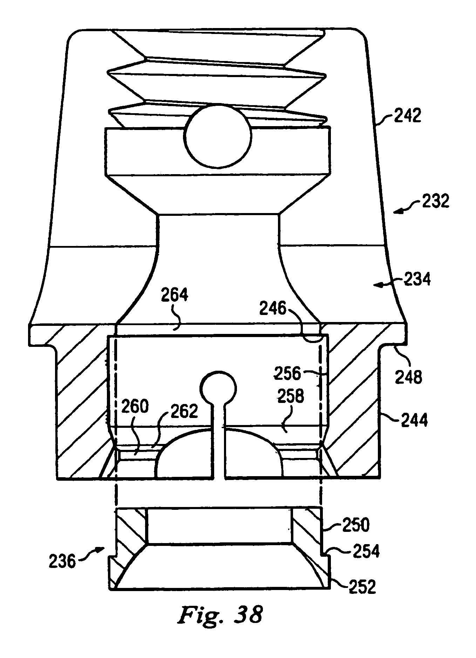

Referring now to Figs. 36-38, shown therein is a bone anchor assembly 232 according to another embodiment of the present disclosure. Fig. 36 is a diagrammatic exploded perspective view of the bone anchor assembly 232; Fig. 37 is a diagrammatic front cross-sectional view of the bone anchor assembly 232; Fig. 38 is a diagrammatic front cross-sectional view of a saddle member 234 and a crown member 236 of the bone anchor assembly 232. The bone anchor assembly 232 includes the receiver or saddle 234, the crown washer 236, a bone anchor 238, and a sleeve 240. In some aspects the bone anchor assembly 232 and its components, the saddle 234, the crown 236, the bone anchor 238, and the sleeve 240, may be substantially similar to the bone anchor assemblies and components described above. Therefore, only some aspects of the bone anchor assembly 232 will be described in detail.

The saddle member 234 generally has a U-shape with an upper portion 242 and a lower portion 244. The upper portion 242 is configured to receive an elongated member, such as rod 24 described above, and also configured to be threadingly coupled with a set screw, such as set screw 22 describe above. The lower portion 244 of the saddle member 234 has a reduced diameter compared to the upper portion 242. Accordingly, an inner shoulder 246 and an outer shoulder 248 are defined between the upper portion 242 and the lower portion 244. The inner shoulder 246 is provided to act as a stop for the crown member 236. Generally, the inner shoulder 246 serves as an upper boundary for the crown member 236 within the saddle 234, preventing upward movement of the crown beyond the shoulder. In the current embodiment, the inner shoulder 246 is positioned below the u-shaped channels of the upper portion. As shown, the crown member 236 includes an upper portion 250, a lower portion 252, and a shoulder 254. The upper portion 250 has a reduced diameter compared to the lower portion 252 and is sized such that the upper portion may extend upwards beyond the inner shoulder 246 of the saddle 214 to engage with the rod 24. In that regard, the shoulder 254 of the crown member 236 engages with the inner shoulder 246 of the saddle 234 to limit the upward translation of the crown member 236 within the saddle.

Referring more specifically to Fig. 38, the lower portion 244 includes an inner surface 256 configured to receive at least the head of a bone anchor. In that regard, the inner surface 256 includes a tapered portion 258 extending inwardly from the inner surface to a surface 260 defined by a cylindrical bore, the intersection of the tapered portion 258 and the surface 260 defining an edge 262. In some embodiments, the tapered portion 258 has a substantially conical shape. The surface 260 defines the narrowest opening in the lower portion 244 of a bore extending through the saddle 234 along the longitudinal axis of the saddle. In the current embodiment, the diameter of the bore defining the surface 260 is substantially similar to the diameter of a bore defining a surface 264 adjacent the inner shoulder 246 of the saddle 234. The diameter of the upper portion 250 of the crown 236 is sized such that the upper portion may pass through the bores defined by the surfaces 260, 264. In other embodiments, especially where the surfaces 260, 264 and/or the upper portion 250 of the crown 236 are not cylindrical, the dimensions of each of these components may be sized to work in a similar manner, but with different shapes. Accordingly, in some embodiments the outer dimension of the upper portion of the crown is sized such that it may pass through the openings defined by inner surfaces of the saddle 34.

In the current embodiment, the diameter of the upper portion 250 of the crown 236 is substantially similar to or slightly less than the diameter of the bores defining the surfaces 260, 264. In that regard, in some embodiments the upper portion 250 of the crown 236 may be positioned within the bore defining surface 260 to align the crown with the opening. Once the crown 236 is aligned with the opening in the saddle 234, the lower portion 252 of the crown is advanced into and through the opening—thereby expanding or flexing the lower portion 244 of the saddle—until the crown is positioned entirely within the saddle. In some embodiments the crown 236 includes a tapered surface extending between the upper portion and the lower portion to facilitate introduction of the crown into the saddle 234. In such embodiments, the tapered surface may be in addition to the shoulder 254 or in lieu of the shoulder.

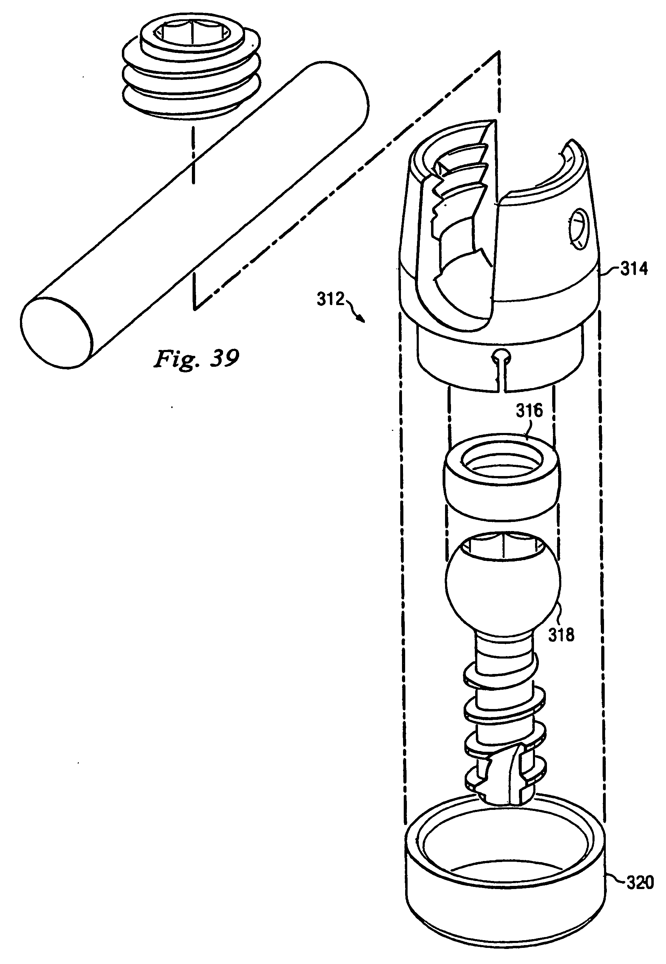

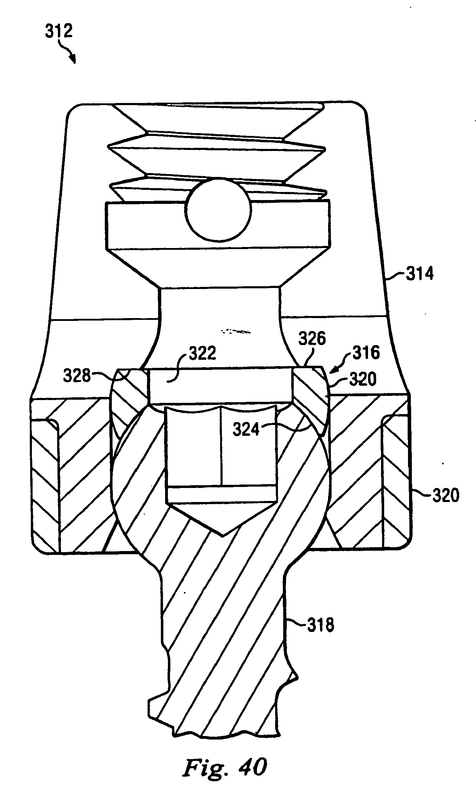

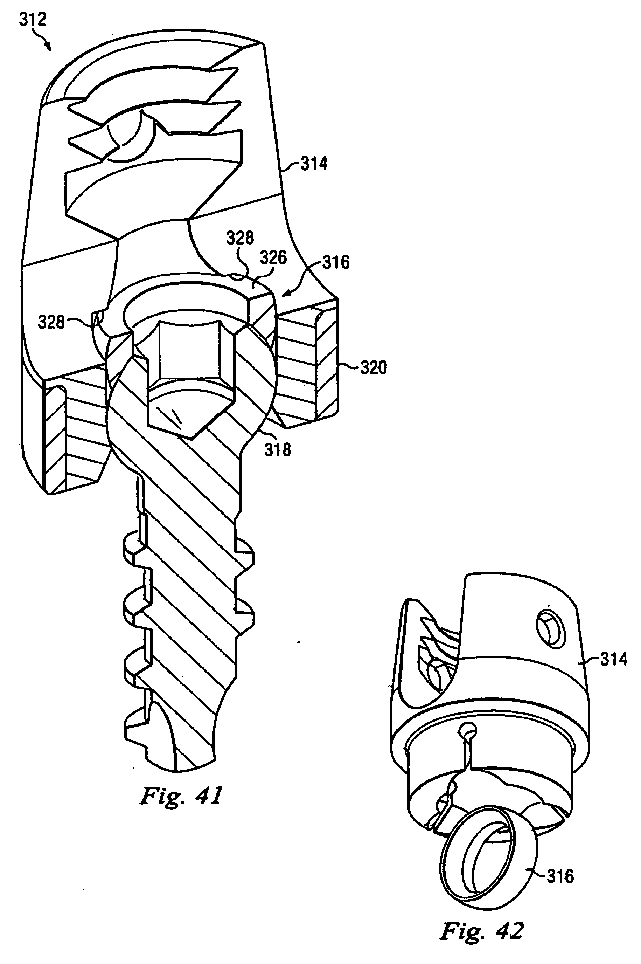

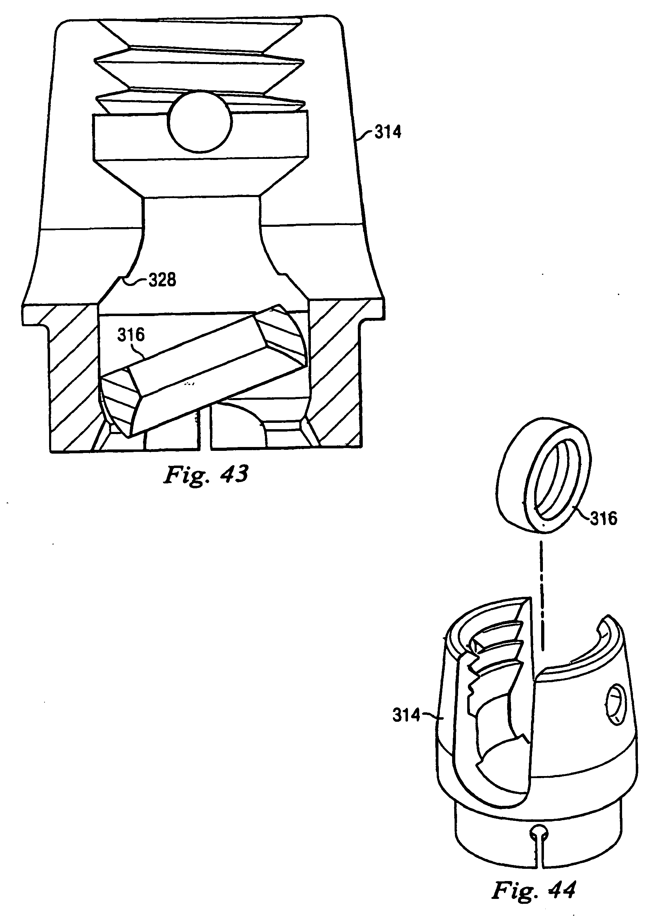

Referring now to Figs. 39-43, shown therein is a bone anchor assembly 312 according to another embodiment of the present disclosure. Fig. 39 is a diagrammatic exploded perspective view of the bone anchor assembly 312; Fig. 40 is a diagrammatic front cross-sectional view of the bone anchor assembly 312; Fig. 41 is a diagrammatic perspective cross-sectional view of the bone anchor assembly 312; Fig. 42 is a diagrammatic perspective view of a saddle member 314 and a crown member 316 of the bone anchor assembly 312; Fig. 43 is a diagrammatic front cross-sectional view of the saddle member 314 and the crown member 316. In some aspects the bone anchor assembly 312 and its components, the saddle 314, the crown 316, the bone anchor 318, and the sleeve 320, may be substantially similar to the bone anchor assemblies and components described above. Therefore, only some aspects of the bone anchor assembly 312 will be described in detail.

Referring more specifically to Figs. 40 and 41, the crown member 316 has a rounded outer surface 320, a substantially cylindrical inner surface 322, an undersurface 324, and an upper surface 326. Crown member 316 is sized to fit within the saddle 314 such that crown member has some axial and rotational freedom of movement therein. For example, crown member 316 is sized to move axially between a shoulder 328 of the saddle 314 and the head of the bone anchor 318. Further, in some embodiments crown member 316 is sized such that it may be rotated within saddle 314 to facilitate insertion of the crown member therein. In that regard, the outer surface 320 may be partially spherical in shape. In some embodiments, the outer surface 320 has a radius of curvature substantially similar to the radius of curvature of the outer surface of the head of the bone anchor 318.

Undersurface 324 is configured to accommodate at least a portion of the head of the bone anchor 318. The undersurface 324 may be shaped (e.g. spherical, rounded, conical, or otherwise) to allow relative multi-axial movement between the crown and the head of the bone anchor 318. In the current embodiment, the undersurface 324 is partially rounded or spherical to mate with the spherical head of the bone anchor. In that regard, the rounded portion of the undersurface 324 has substantially the same radius of curvature as the head of the bone anchor 318. The undersurface 324 is shaped such that sufficient compression of the head of the bone anchor 318 between the crown member 316 and the seat of the saddle 314 can fixedly secure the bone anchor relative to the saddle. The upper surface 328 of the crown member 316 is configured to engage with the an elongated member, such as spinal rod 24. In particular, the upper surface 328 is configured such that the crown member 316 may be compressed downwardly by an elongated member to secure the bone anchor 318 in place.

Referring now to Figs. 42 and 43, in some aspects the crown member 316 is bottom-loaded into the saddle 314. In some embodiments, the crown member 316 is loaded such that the saddle 314 is not expanded. For example, as shown in Fig. 42 the crown member 316 is oriented such that the cylindrical bore defined by the inner surface 322 is substantially transverse to a longitudinal axis of the saddle 314. Then, utilizing at least a portion of at least one of the cutouts in the bottom portion of the saddle 314, the crown member 316 is inserted through the bottom of the saddle without expanding the saddle. As best seen in Fig. 40, the outer diameter of the crown member 316 is greater than the narrowest part of the bore in the lower portion of the saddle 314. Accordingly, the crown member 316 cannot be inserted with the cylindrical bore defined by the inner surface 322 being substantially parallel with the longitudinal axis of the saddle 314 without expanding the saddle. Once positioned within the saddle 314, the crown member 316 is rotated approximately 90 degrees with respect to the longitudinal axis of the saddle. As shown in Fig. 43, the outer diameter of the crown member 316 is such that it may be rotated within the saddle 314. In that regard and as mentioned above, in some embodiments the outer diameter of the crown member 316 may be substantially similar to that of the head of the bone anchor 318. After being rotated within the saddle, the crown member 316 is then movably held therein by the shoulder 328 and the lower portion of the saddle.

Referring now to Fig. 44, in some aspects the crown member 316 is top-loaded into the saddle 314. As shown, the crown member 316 is oriented such that the cylindrical bore defined by the inner surface 322 is substantially transverse to a longitudinal axis of the saddle 314. Further, the crown member 316 is aligned such that the crown member may be lowered into the saddle without interference from the portion of the saddle defining the shoulder 328. In the current embodiment, the crown member 316 is substantially aligned with the channels defined in the upper portion of the saddle. Then, the crown member 316 is inserted through the top of the saddle and into the lower portion of the saddle. Once positioned within lower portion of the saddle 314, the crown member 316 is rotated approximately 90 degrees with respect to the longitudinal axis of the saddle. Again, as shown in Fig. 43, the outer diameter of the crown member 316 is such that it may be rotated within the saddle 314. After being rotated within the saddle, the crown member 316 is then movably held therein by the shoulder 328 and the lower portion of the saddle.

While the several embodiments of the disclosure have been illustrated and described in detail in the drawings and foregoing description, this is to be considered as illustrative and not restrictive in character, it being understood that all changes and modifications that come within the disclosure are desired to be protected. For example, it is fully contemplated that the features described with respect to one embodiment may be selectively combined with the features of other embodiments.

The foregoing outlines features of several embodiments so that those skilled in the art may better understand the aspects of the present disclosure. Those skilled in the art should appreciate that they may readily use the present disclosure as a basis for designing or modifying other processes and structures for carrying out the same purposes and/or achieving the same advantages of the embodiments introduced herein. Those skilled in the art should also realize that such equivalent constructions do not depart from the scope of the present disclosure, and that they may make various changes, substitutions and alterations herein without departing from the scope of the present disclosure.

## Claims

1. A bone anchor assembly (12; 112; 212; 232; 312), comprising:
a bone anchor (18; 118; 218; 238; 318) having a head portion and a bone engaging portion;
a crown member (16; 116; 216; 236; 316) shaped and sized to mate with the head portion of the bone anchor (18; 118; 218; 238; 318);
a saddle (14; 114; 214; 234; 314) having an upper portion (26; 126; 222) and a lower portion (28; 128; 224),
the upper portion (26; 126; 222) having a channel (32; 132) for receiving an elongated members (24) and a threaded portion (46; 146) for receiving a compression member (22),
the lower portion (28; 128; 224) configured to receive the crown member (16; 116; 216; 236; 316) and the head portion of the bone anchor (18; 118; 218; 238; 318) therein, the lower portion (28; 128; 224) including a plurality of slots (60) rendering the lower portion (28; 128; 224) outwardly flexible to receive the head portion of the bone anchor (18; 118; 218; 238; 318) and inwardly flexible to secure the head portion of the bone anchor (18; 118: 218; 238; 318) therein; and
a sleeve (20; 120; 220; 240; 320) configured for positioning around the lower portion (28; 128; 224) of the saddle (14; 114; 214; 234; 314), the sleeve (20, 120; 220; 240; 320) sized such that the lower portion (28; 128; 224) of the saddle (14; 114, 214; 234; 314) secures the head portion of the bone anchor (18; 118; 218; 238; 318) therein upon advancement of the sleeve (20; 120; 220; 240; 320) upwardly around the lower portion (28; 128; 224) of the saddle (14; 114; 214; 234; 314), the sleeve (20; 120; 220; 240; 320) thereby preventing the lower portion (28; 128; 224) of the saddle (14; 114; 214; 234; 314) from flexing outwardly;
wherein the bone anchor (18; 118; 218; 238; 318) is moveable with respect to the saddle (14; 114; 214; 234; 314) when the sleeve (20; 120; 220; 240; 320) is positioned around the lower portion (28; 128; 224); and
wherein the bone anchor (18; 118; 218; 238; 318) is secured in a fixed position relative to the saddle (14; 114; 214; 234; 314) by compression of the head portion of the bone anchor (18; 118; 218; 238; 318) between the crown member (16; 116; 216; 236; 316) and the lower portion (28; 128; 224) of the saddle (14; 114; 214; 234) created by threadingly advancing the compression member against the elongated member (24), wherein the lower portion (28) comprises a seat portion for interfacing with the head portion of the bone anchor, wherein the upper portion (26) further comprises a shoulder (50) for limiting the upward translation of the crown member (16), and wherein the shoulder (50) limiting the upward translation is positioned above a bottom portion of the channel (32) for receiving the elongated member (24).

2. The assembly (12; 112; 212; 232; 312) of claim 1, wherein the seat is preferably configured to transmit forces from the head portion of the bone anchor (18) radially outward to the sleeve (20) when the sleeve is positioned around the lower portion (28) of the saddle (14).

3. The assembly (12; 112; 212; 232; 312) of claim 2, wherein the seat comprises a tapered surface having an angle between 10 and 80 degrees, preferably an angle of approximately 20 degrees or of approximately 55 degrees, relative to a longitudinal axis of the saddle (14).

4. The assembly (12; 112; 212; 232; 312) of claim 1, wherein the saddle (14) further comprises an external shoulder (52) for limiting the upward translation of the sleeve about the lower potion (28) of the saddle (14).

5. The assembly (12; 112; 212; 232; 312) of claim 1, wherein at least one of the lower portion (28) of the saddle (14) and the sleeve (20) includes a chamfer (94) for guiding the sleeve (20) around the lower portion (28).

6. The assembly (12; 112; 212; 232; 312) of claim 1, therein the sleeve (20) comprises a first material and the lower portion of the saddle comprises a second material, the first material having at least one property-different than the second material.

7. The assembly (12; 112; 212; 232; 312) of claim 1, wherein the plurality of slots (60) extend upwardly from the bottom of the lower portion of the saddle and include a relieve having an increased width relative to the remainder of the slot at an upper end thereof.

8. The assembly (12; 112; 212; 232; 312) of claim 6, wherein the lower portion (28; 128) further comprises a plurality of cutout portions (72; 172) configured to allow an increased range of motion between the saddle (14; 114) and the bone anchor (18).

9. The assembly (12; 112; 212; 232; 312) of claim 7, wherein the sleeve (120) includes a plurality of outer cutout portions (114) configured to allow an increased range of motion between the saddle (114) and the bone anchor (18).

10. The assembly (12; 112; 212; 232; 312) of claim 7, wherein the plurality of slots are substantially aligned with the plurality of cutouts (72; 172) of the lower portion (28; 128).

11. A system for orthopaedic implantation, comprising. an elongated member (24), and
bone anchor assembly (12) according to any one of claims 1 to 9.

## Patentansprüche

1. Eine Knochenankervorrichtung (12, 112, 212, 232, 312), aufweisend:
einen Knochenanker (18, 118, 218, 238, 318), der einen Kopfabschnitt und einen Knocheneingriffsabschnitt aufweist,
ein Kronenelement (16, 116, 216, 236, 316), das geformt und bemessen ist, um zu dem Kopfabschnitt des Knochenankers (18, 118, 218, 238, 318) zu passen,
einen Sattel (14, 114, 214, 234, 314), der einen oberen Abschnitt (26, 126, 222) und einen unteren Abschnitt (28, 128, 224) aufweist,
wobei der obere Abschnitt (26, 126, 222) aufweist: einen Kanal (32, 132), um ein langgestrecktes Element (24) aufzunehmen, und einen Gewindeabschnitt (46, 146), um ein Kompressionselement (22) aufzunehmen,
wobei der untere Abschnitt (28, 128, 224) eingerichtet ist, um das Kronenelement (16, 116, 216, 236, 316) und den Kopfabschnitt des Knochenankers (18, 118, 218, 238, 318) darin aufzunehmen, wobei der untere Abschnitt (28, 128, 224) eine Mehrzahl von Schlitzen (60) aufweist, die den unteren Abschnitt (28, 128, 224) nach außen flexibel machen, um den Kopfabschnitt des Knochenankers (18, 118, 218, 238, 318) aufzunehmen, und nach innen flexibel machen, um den Kopfabschnitt des Knochenankers (18, 118, 218, 238, 318) darin zu sichern, und
eine Hülse (20, 120, 220, 240, 320), die zum Positionieren um den unteren Abschnitt (28, 128, 224) des Sattels (14, 114, 214, 234, 314) herum eingerichtet ist, wobei die Hülse (20, 120, 220, 240, 430) derart bemessen ist, dass der untere Abschnitt (28, 128, 224) des Sattels (14, 114, 214, 234, 314) den Kopfabschnitt des Knochenankers (18, 118, 218, 238, 318) bei Vorrücken der Hülse (20, 120, 220, 240, 320) um den unteren Abschnitt (28, 128, 224) des Sattels (14, 114, 214, 234, 314) nach oben darin sichert, wobei die Hülse (20, 120, 220, 240, 320) dadurch verhindert, dass sich der untere Abschnitt (28, 128, 224) des Sattels (14, 114, 214, 234, 314) nach außen biegt,
wobei der Knochenanker (18, 118, 218, 238, 318) bezüglich des Sattels (14, 114, 214, 234, 314) bewegbar ist, wenn die Hülse (20, 120, 220, 240, 320) um den unteren Abschnitt (28, 128, 224) herum positioniert ist, und
wobei der Knochenanker (18, 118, 218, 238, 318) durch durch schraubenartiges Vorrücken des Kompressionselements gegen das langgestreckte Element (24) erzeugtes Zusammendrücken des Kopfabschnitts des Knochenankers (18, 118, 218, 238, 318) zwischen dem Kronenelement (16, 116, 216, 236, 316) und dem unteren Abschnitt (28, 128, 224) des Sattels (14, 114, 214, 234) in einer festen Position bezüglich des Sattels (14, 114, 214, 234, 314) gesichert wird, wobei der untere Abschnitt (28) einen Sitzabschnitt für ein Verbinden mit dem Kopfabschnitt des Knochenankers aufweist, wobei der obere Abschnitt (26) ferner eine Schulter (50) aufweist, um die Aufwärtstranslation des Kronenelements (16) zu begrenzen, und wobei die Schulter (50), die die Aufwärtstranslation begrenzt, über einem Bodenabschnitt des Kanals (32) zum Aufnehmen des langgestreckten Elements (24) angeordnet ist.

2. Die Vorrichtung (12, 112, 212, 232, 312) gemäß Anspruch 1, wobei der Sitz vorzugsweise eingerichtet ist, um Kräfte von dem Kopfabschnitt des Knochenankers (18) aus radial nach außen an die Hülse (20) zu übertragen, wenn die Hülse um den unteren Abschnitt (28) des Sattels (14) herum positioniert ist.

3. Die Vorrichtung (12, 112, 212, 232, 312) gemäß Anspruch 2, wobei der Sitz eine konisch zulaufende Fläche aufweist, die bezüglich einer Längsachse des Sattels (14) einen Winkel von zwischen 10 und 80 Grad, vorzugsweise einen Winkel von ungefähr 20 Grad oder von ungefähr 55 Grad aufweist.

4. Die Vorrichtung (12, 112, 212, 232, 312) gemäß Anspruch 1, wobei der Sattel (14) ferner eine äußere Schulter (52) aufweist, um die Aufwärtstranslation der Hülse um den unteren Abschnitt (28) des Sattels (14) zu begrenzen.

5. Die Vorrichtung (12, 112, 212, 232, 312) gemäß Anspruch 1, wobei mindestens einer von dem unteren Abschnitt (28) des Sattels (14) und der Hülse (20) eine Abfasung (94) aufweist, um die Hülse (20) um den unteren Abschnitt (28) zu führen.

6. Die Vorrichtung (12, 112, 212, 232, 312) gemäß Anspruch 1, wobei die Hülse (20) ein erstes Material aufweist und der untere Abschnitt des Sattels ein zweites Material aufweist, wobei das erste Material mindestens eine Eigenschaft hat, die sich von dem zweiten Material unterscheidet.

7. Die Vorrichtung (12, 112, 212, 232, 312) gemäß Anspruch 1, wobei sich die Mehrzahl von Schlitzen (60) von dem Boden des unteren Abschnitts des Sattels in Richtung nach oben erstreckt und eine Aussparung aufweist, die bezüglich des Restes des Schlitzes an einem oberen Ende davon eine vergrößerte Breite hat.

8. Die Vorrichtung (12, 112, 212, 232, 312) gemäß Anspruch 6, wobei der untere Abschnitt (28, 128) ferner eine Mehrzahl von Ausschnittabschnitten (72, 172) aufweist, die eingerichtet sind, um zwischen dem Sattel (14, 114) und dem Knochenanker (18) einen vergrößerten Bewegungsbereich zu ermöglichen.

9. Die Vorrichtung (12, 112, 212, 232, 312) gemäß Anspruch 7, wobei die Hülse (120) eine Mehrzahl von äußeren Ausschnittabschnitten (174) aufweist, die eingerichtet sind, um zwischen dem Sattel (114) und dem Knochenanker (18) einen vergrößerten Bewegungsbereich zu ermöglichen.

10. Die Vorrichtung (12, 112, 212, 232, 312) gemäß Anspruch 7, wobei die Mehrzahl von Schlitzen im Wesentlichen zu der Mehrzahl von Ausschnitten (72, 172) des unteren Abschnitts (28, 128) ausgerichtet ist.

11. Ein System zur orthopädischen Implantation, aufweisend:
ein langgestrecktes Element (24) und
die Knochenankervorrichtung (12) gemäß irgendeinem der Ansprüche 1 bis 9.

## Revendications

1. Assemblage d'ancrage osseux (12 ; 112 ; 212 ; 232 ; 312), comprenant :
un ancrage osseux (18 ; 118 ; 218 ; 238 ; 318) ayant une partie de tête et une partie de mise en prise à l'os ;
un élément formant couronne (16 ; 116 ; 216 ; 236 ; 316) formé et dimensionné pour s'apparier avec la partie de tête de l'ancrage osseux (18 ; 118 ; 218 ; 238 ; 318) ;
une selle (14 ; 114 ; 214 ; 234 ; 314) ayant une partie supérieure (26 ; 126 ; 222) et une partie inférieure (28 ; 128 ; 224),
la partie supérieure (26 ; 126 ; 222) ayant un canal (32 ; 132) pour recevoir un élément allongé (24) et une partie filetée (46 ; 146) pour recevoir un élément de compression (22),
la partie inférieure (28 ; 128 ; 224) étant configurée pour recevoir l'élément formant couronne (16 ; 116 ; 216 ; 236 ; 316) et la partie de tête de l'ancrage osseux (18 ; 118 ; 218 ; 238 ; 318) à l'intérieur de celle-ci, la partie inférieure (28 ; 128 ; 224) comprenant une pluralité de fentes (60) rendant la partie inférieure (28 ; 128 ; 224) flexible vers l'extérieur pour recevoir la partie de tête de l'ancrage osseux (18 ; 118 ; 218 ; 238 ; 318) et flexible vers l'intérieur pour immobiliser la partie de tête de l'ancrage osseux (18 ; 118 ; 218 ; 238 ; 318) à l'intérieur de celle-ei ; et
un manchon (20 ; 120 ; 220 ; 240 ; 320) configuré pour le positionnement autour de la partie inférieure (28 ; 128 ; 224) de la selle (14 ; 114 ; 214 ; 234 ; 314), le manchon (20 ; 120 ; 220 ; 240 ; 320) étant dimensionné de sorte que la partie inférieure (28 ; 128 ; 224) de la selle (14 ; 114 ; 214 ; 234 ; 314) fixe la partie de tête de l'ancrage osseux (18 ; 118 ; 218 ; 238 ; 318) à l'intérieur de celle-ci lors de l'avancement du manchon (20 ; 120 ; 220 ; 240 ; 320) vers le haut autour de la partie inférieure (28 ; 128 ; 224) de la selle (14; 114 ; 214 ; 234 ; 314), le manchon (20 ; 120 ; 220 ; 240 ; 320) empêchant ainsi la partie inférieure (28 ; 128 ; 224) de la selle (14 ; 114 ; 214 ; 234 ; 314) de se courber vers l'extérieur ;
dans lequel l'ancrage osseux (18 ; 118 ; 218 ; 238 ; 318) est mobile par rapport à la selle (14 ; 114 ; 214 ; 234 ; 314) quand le manchon (20 ; 120 ; 220 ; 240 ; 320) est positionné autour de la partie inférieure (28 ; 128 ; 224) ; et
dans lequel l'ancrage osseux (18 ; 118 ; 218 ; 238 ; 318) est immobilisé dans une position fixe par rapport à la selle (14 ; 114 ; 214 ; 234 ; 314) par compression de la partie de tête de l'ancrage osseux (18 ; 118 ; 218 ; 238 ; 318) entre l'élément formant couronne (16 ; 116 ; 216 ; 236 ; 316) et la partie inférieure (28 ; 128 ; 224) de la selle (14 ; 114 ; 214 ; 234) provoquée en faisant avancer par vissage l'élément de compression contre l'élément allongé (24), dans lequel la partie inférieure (28) comprend une partie de siège pour jouer le rôle d'interface avec la partie de tête de l'ancrage osseux, dans lequel la partie supérieure (26) comprend en outre un épaulement (50) pour limiter la translation vers le haut de l'élément formant couronne (16), et dans lequel l'épaulement (50) limitant la translation vers le haut est positionné au-dessus d'une partie de fond du canal (32) pour recevoir l'élément allongé (24).

2. Assemblage (12 ; 112 ; 212 ; 232 ; 312) selon la revendication 1, dans lequel le siège est configuré de préférence pour transmettre des forces à partir de la partie de tête de l'ancrage osseux (18) radialement vers l'extérieur au manchon (20) quand le manchon est positionné autour de la partie inférieure (28) de la selle (14).

3. Assemblage (12 ; 112 ; 212 ; 232 ; 312) selon la revendication 2, dans lequel le siège comprend une surface effilée ayant un angle compris entre 10 et 80 degrés, de préférence un angle d'approximativement 20 degrés ou d'approximativement 55 degrés par rapport à un axe longitudinal de la selle (14).

4. Assemblage (12 ; 112 ; 212 ; 232 ; 312) selon la revendication 1, dans lequel la selle (14) comprend en outre un épaulement externe (52) pour limiter la translation vers le haut du manchon autour de la partie inférieure (28) de la selle (14).

5. Assemblage (12 ; 112 ; 212 ; 232 ; 312) selon la revendication 1, dans lequel au moins l'un parmi la partie inférieure (28) de la selle (14) et le manchon (20) comprend un chanfrein (94) pour guider le manchon (20) autour de la partie inférieure (28).

6. Assemblage (12 ; 112 ; 212 ; 232 ; 312) selon la revendication 1, dans lequel le manchon (20) comprend un premier matériau et la partie inférieure de la selle comprend un deuxième matériau, le premier matériau ayant au moins une propriété différente du deuxième matériau.

7. Assemblage (12 ; 112 ; 212 ; 232 ; 312) selon la revendication 1, dans lequel la pluralité de fentes (60) s'étend vers le haut à partir du fond de la partie inférieure de la selle et comprend une dépouille ayant une largeur augmentée par rapport au reste de la fente à une extrémité supérieure de celle-ci.

8. Assemblage (12 ; 112; 212 ; 232 ; 312) selon la revendication 6, dans lequel la partie inférieure (28 ; 128) comprend en outre une pluralité de parties découpées (72 ; 172) configurées pour permettre une plage de déplacement plus grande entre la selle (14 ; 114) et l'ancrage osseux (18).

9. Assemblage (12 ; 112 ; 212 ; 232 ; 312) selon la revendication 7, dans lequel le manchon (120) comprend une pluralité de parties découpées extérieures (174) configurées pour permettre une plage de déplacement plus grande entre la selle (114) et l'ancrage osseux (18).

10. Assemblage (12 ; 112 ; 212 ; 232 ; 312) selon la revendication 7, dans lequel la pluralité de fentes sont sensiblement alignées avec la pluralité de découpes (72 ; 172) de la partie inférieure (28 ; 128).

11. Système d'implantation orthopédique, comprenant :
un élément allongé (24), et
un assemblage d'ancrage osseux (12) selon l'une quelconque des revendications 1 à9.
